(19) Europäisches Patentamt
European Patent Office
Office européen des brevets

(11) Veröffentlichungsnummer: **0 324 347 A2**

(12) **EUROPÄISCHE PATENTANMELDUNG**

(21) Anmeldenummer: **89100031.7**

(22) Anmeldetag: **03.01.89**

(51) Int. Cl.4: **C07D 237/08 , C07D 237/24 , C07D 405/06 , A61K 31/50**

Patentansprüche für folgende Vertragsstaaten: ES + GR.

(30) Priorität: **09.01.88 DE 3800439**
**14.01.88 DE 3800785**

(43) Veröffentlichungstag der Anmeldung: **19.07.89 Patentblatt 89/29**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Baader, Ekkehard, Dr.**
**Amselweg 14**
**D-6240 Königstein/Taunus(DE)**
Erfinder: **Jendralla, Heiner, Dr.**
**Pfortengartenweg 38**
**D-6230 Frankfurt am Main 80(DE)**
Erfinder: **Kerekjarto, Bela, Dr.**
**Weilbächer Wälder FA8**
**D-6238 Hofheim am Taunus(DE)**
Erfinder: **Beck, Gerhard, Dr.**
**Gustav-Freytag-Strasse 24**
**D-6000 Frankfurt am Main(DE)**

(54) **Neue 3,5-Dihydroxycarbonsäuren und deren Derivate, Verfahren zu ihrer Herstellung, ihre Verwendung als Arzneimittel, pharmazeutische Präparate und Zwischenprodukte.**

(57) 3,5-Dihydroxycarbonsäuren und deren Derivate der Formel I

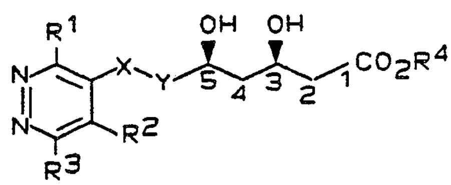

und die entsprechenden Laktone der Formel II

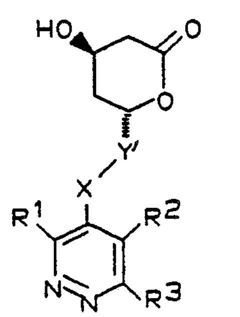

II

worin $R^1$, $R^2$, $R^3$, $R^4$ und X-Y die angegebenen Bedeutungen haben, Verfahren zur Herstellung dieser Verbindungen, ihre Verwendung als Arzneimittel und pharmazeutische Präparate werden beschrieben.

EP 0 324 347 A2

Außerdem werden neue Zwischenprodukte für die Herstellung der Verbindungen der Formeln I und II beschrieben.

# Neue 3,5-Dihydroxycarbonsäuren und deren Derivate, Verfahren zu ihrer Herstellung, ihre Verwendung als Arzneimittel, pharmazeutische Präparate und Zwischenprodukte

Das Enzym 3-Hydroxy-3-methylglutaryl-Coenzym-A-Reduktase (HMG-CoA-Reduktase) katalysiert die Bildung von Mevalonsäure aus 3-Hydroxy-3-methylglutaryl-Coenzym A (HMG-CoA). Diese Reaktion spielt eine zentrale Rolle bei der Biosynthese des Cholesterins. Derivate der 3-Hydroxy-3-methyl-glutarsäure (HMG) und der Mevalonsäure sind als Hemmer der Cholesterinbiosynthese beschrieben worden (M.R. Boots et al., J. Pharm. Sci. 69, 306 (1980), F.M. Singer et al., Proc. Soc. Exper. Biol. Med. 102, 370 (1959), H. Feres, Tetrahedron Lett. 24, 3769 (1983)). 3-Hydroxy-3-methylglutarsäure selbst zeigt an der Ratte und im Humanversuch signifikante cholesterinsenkende Wirkung (Z. Beg, Experientia 23, 380 (1967), ibid 24, 15 (1968), P. J. Lupien et al., Lancet 1979, 1, 283).

Es wurde nun gefunden, daß Dihydroxycarbonsäuren der allgemeinen Formel I sowie die entsprechenden Laktone der Formel II Hemmstoffe der HMG-CoA-Reduktase sind.

Die Erfindung betrifft daher, 3,5-Dihydroxycarbonsäuren und deren Derivate der allgemeinen Formel I

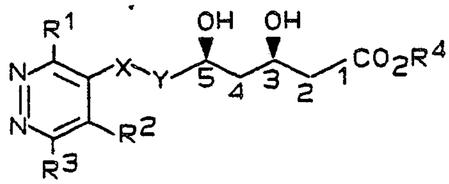

I

und die entsprechenden Laktone der Formel II

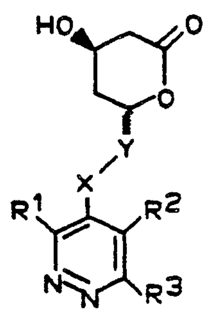

II

In den allgemeinen Formeln I und II bedeuten

X-Y einen Rest der Formel -CH=CH- oder $-CH_2-CH_2-$

$R^1$, $R^2$, $R^3$ unabhängig voneinander Wasserstoff, einen gesättigten oder ungesättigten, geradkettigen oder verzweigten Kohlenwasserstoffrest mit bis zu 6 C-Atomen, der gegebenenfalls am terminalen Kohlenstoff durch einen gesättigten oder ungesättigten, cyclischen Kohlenwasserstoffrest mit 3 bis 6 C-Atomen, substituiert sein kann,

einen cyclischen gesättigten oder bis zu zweifach ungesättigten Kohlenwasserstoffrest mit 3 bis 7 C-Atomen, einen aromatischen Rest ausgewählt aus der Gruppe Phenyl, Furyl, Thienyl, Pyridyl, der gegebenenfalls im Kern 1 bis 3 gleiche oder verschiedene Substituenten der folgenden Gruppen tragen kann:

Halogen, Trifluormethyl, Alkyl oder Alkenyl mit je bis zu 6 C-Atomen, Hydroxy, Alkoxy mit 1 bis 6 C-Atomen, Carboxy, Carbalkoxy mit 1 bis 6 C-Atomen im Alkoxyteil

$R^4$ Wasserstoff, einen geradkettigen oder verzweigten, gesättigten oder ungesättigten Kohlenwasserstoffrest mit bis zu 8 C-Atomen, Mono- oder Dihydroxyalkyl mit 1 bis 4 C-Atomen, einen Phenyl- oder Benzylrest, deren Kerne 1- bis 2-fach mit Halogen oder einem Alkylrest mit 1 bis 4 C-Atomen substituiert sein können, Alkalimetall oder ein Ammoniumion.

Unter den Substituenten $R^1$ und $R^2$ sind bevorzugt ein geradkettiger oder verzweigter Alkylrest mit 1 bis 4 C-Atomen, ein Cycloalkylrest mit 5 bis 6 C-Atomen, ein Cycloalkylmethyl- oder Cycloalkenylmethylrest mit einer Ringgröße von 5 bis 6 C-Atomen, ein Phenylrest, der gegebenenfalls 1 bis 3 gleiche oder verschiedene Substituenten der folgenden Gruppen tragen kann Halogen, Trifluormethyl, Alkyl mit 1 bis 4 C-Atomen, Hydroxy, Alkoxy mit 1 bis 4 C-Atomen oder Carbalkoxy mit 1 bis 4 C-Atomen im Alkoxyteil.

Unter den Bedeutungen für $R^3$ sind bevorzugt Wasserstoff, ein geradkettiger ode verzweigter Alkyl-

oder Alkenylrest mit bis zu 6 C-Atomen, ein Cycloalkyl- oder Cycloalkenylrest mit je 5 bis 6 C-Atomen, ein Phenyl- oder Pyridylrest, wobei die aromatischen Reste gegebenenfalls 1 bis 3 gleiche oder verschiedene Substituenten der folgenden Gruppen tragen können: Halogen, Alkyl mit 1 bis 4 C-Atomen, Hydroxy, Alkoxy mit 1 bis 4 C-Atomen oder Carbalkoxy mit 1 bis 4 C-Atomen im Alkoxyteil.

Unter den Resten $R^4$ sind bevorzugt Wasserstoff, Methyl, Ethyl, Isopropyl, Isobutyl, Benzyl, Natrium, Kalium, Ammonium ($NH_4$) oder Methyltris(hydroxymethyl)ammonium.

Unter den Substituenten $R^1$ sind besonders bevorzugt Methyl, Isopropyl, sek.-Butyl, tert.-Butyl, Cyclohexyl, Phenyl, 4-Chlorphenyl, 4-Fluorphenyl, 4-Methoxyphenyl, 4-Fluor-3-methylphenyl, 3,5-Dimethylphenyl, Cyclohexylmethyl, 4-Trifluormethylphenyl.

Unter den Substituenten $R^2$ sind besonders bevorzugt Methyl, Isopropyl, sek.-Butyl, tert.-Butyl, Cyclohexyl, Phenyl, 4-Chlorphenyl, 4-Fluorphenyl, 4-Methoxyphenyl, 4-Fluor-3-methylphenyl, 3,5-Dimethylphenyl, Cyclohexylmethyl, 4-Trifluormethylphenyl,

Unter den Substituenten $R^3$ sind besonders bevorzugt Wasserstoff, Methyl, Isopropyl, sek.-Butyl, tert.-Butyl, Cyclohexyl, Phenyl, 4-Fluorphenyl, 2,5-Dimethylphenyl, 3,5-Dimethylphenyl, 4-Trifluormethylphenyl.

Unter den Substituenten $R^4$ sind besonders bevorzugt Wasserstoff, Methyl, Ethyl, Natrium, Kalium.

Die Erfindung betrifft die reinen Enantiomeren sowie die Racemate der Formel I und Mischungen aus diesen, also die Racemate mit der absoluten Konfiguration 3R/5S bzw. 3S/5R für X-Y gleich HC=CH und 3R/5R bzw. 3S/5S für X-Y gleich $CH_2$-$CH_2$ sowie die reinen Enantiomeren 3R/5S für X-Y gleich HC=CH und 3R/5R für X-Y gleich $CH_2$-$CH_2$.

Die Erfindung betrifft ferner die reinen Enantiomeren sowie die Racemate der allgemeinen Formel II, die aus den obengenannten stereoisomeren offenkettigen Dihydroxycarbonsäuren der allgemeinen Formel I hervorgehen. Im einzelnen sind das die Racemate mit den absoluten Konfigurationen 3R/5S bzw. 3S/5R für X-Y gleich HC=CH und 3R/5R bzw. 3S/5S für X-Y gleich $CH_2$-$CH_2$ sowie die reinen Enantiomeren 3R/5S für X-Y gleich HC=CH und 3R/5R für X-Y gleich $CH_2$-$CH_2$.

Die Erfindung betrifft ferner ein Verfahren zur Herstellung der Verbindungen der allgemeinen Formeln I und II, das dadurch gekennzeichnet ist, daß man

a) entsprechend substituierte Aldehyde der Formel III

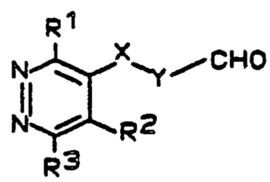

III

worin X-Y, $R^1$, $R^2$ und $R^3$ die angegebenen Bedeutungen haben, in die entsprechenden Hydroxyketoester der allgemeinen Formel IV überführt

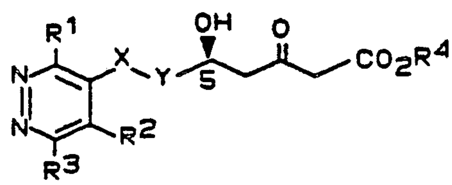

IV

worin X-Y, $R^1$, $R^2$ und $R^3$ die angegebenen Bedeutungen haben und $R^4$ Alkyl mit 1 bis 8 C-Atomen bedeutet,

b) die Hydroxyketoester der Formel IV in die entsprechenden 3,5-Dihydroxyverbindungen der Formel I

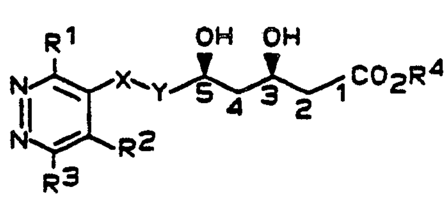

I

worin X-Y, $R^1$, $R^2$ und $R^3$ die zu Formel I angegebenen Bedeutungen haben, und $R^4$ Alkyl mit 1 bis 8 C-Atomen ist, überführt und eine erhaltene Verbindung gegebenenfalls verseift zu einer Verbindung der Formel I, worin $R^4$ ein Alkalimetallkation darstellt, daraus gegebenenfalls die freie Säure ($R^4$ = Wasserstoff) in Freiheit setzt und die freie Säure gegebenenfalls in Verbindungen der Formel I, worin $R^4$ die zu Formel I angegebenen Bedeutungen mit Ausnahme von Wasserstoff hat, überführt,

c) und eine erhaltene Verbindung der Formel I gegebenenfalls in ein Lakton der Formel II überführt,

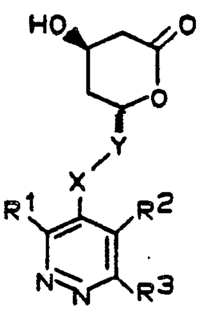

II

worin X-Y, $R^1$, $R^2$ und $R^3$ die angegebenen Bedeutungen haben

d) und eine erhaltene Verbindung, worin X-Y die CH=CH-Gruppe bedeutet, gegebenenfalls hydriert zu einer Verbindung, worin X-Y die $CH_2$-$CH_2$-Gruppe ist.

Die Umwandlung von Verbindungen der Formel III in Verbindungen der Formel IV erfolgt je nach Gegebenheiten und Erfordernissen nach verschiedenen Varianten, wie z.B.

1. Reaktion des Dianions von Acetessigestern mit Aldehyden der Formel III führt in Lösungsmitteln wie THF bei -78° C bis Raumtemperatur zu racemischen Verbindungen der Formel IV. Dianionen von Acetessigestern können mit verschiedenen Basen, vorzugsweise Natriumhydrid und Lithiumdiisopropylamid (LDA) vorzugsweise in THF bei -40° C bis Raumtemperatur bereitet werden.

2. Umsetzung der Enolate von achiralen Essigsäureestern, wie z.B. Ethyl- oder Propylestern, die mit starken Basen, vorzugsweise LDA, in THF bereitet werden, mit Aldehyden der Formel III führt in Lösungsmitteln wie beispielsweise THF bei Temperaturen zwischen -78° C und 0° C zu racemischen Verbindungen der Formel V,

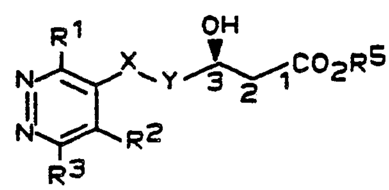

V

worin $R^5$ eine achirale Säureschutzgruppe, wie z.B. die Ethyl- oder Propylgruppe, bedeutet. Umsetzung mit einem weiteren Essigsäureesterenolat führt in Lösungsmitteln wie beispielsweise THF bei -78° C bis Raumtemperatur zu racemischen Verbindungen der Formel IV.

3. Umsetzung von Aldehyden der Formel III mit Lithium-, Natrium-, Kalium- oder Magnesiumenolaten von optisch aktiven Essigsäureestern führt in Lösungsmitteln wie THF bei -78° C bis 0° C zu optisch aktiven Addukten der Formel V. In diesem Fall bedeutet $R^5$ eine geeignete optisch aktive Säureschutzgruppe, die die Stereochemie an 3-C bestimmt. Vorzugsweise wird hier die Gruppe

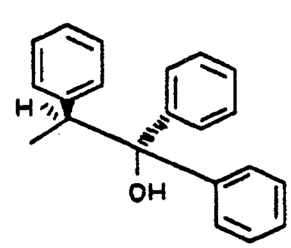

verwendet, die nach M. Braun und R. Devant (Tetrahedron Lett. 25, 5031 (1984)) die 3 R Konfiguration ergibt und aus L-(+)-Mandelsäure hergestellt wird. Es eignen sich aber auch andere chirale optisch aktive Gruppen. Die nun optisch aktiven Verbindungen der Formel V werden mit achiralen Essigsäureesterenolaten nach Variante 2 entweder direkt in die jetzt optisch aktiven Verbindungen der Formel IV überführt oder nach Überführung der primären Addukte in die entsprechenden Alkylester, vorzugsweise Methylester.

Die Umwandlung von Verbindungen der Formel IV in Verbindungen der Formel I erfolgt z.B. in Analogie zu einem literaturbekannten Verfahren (K. Narasaka und H.C. Pai, Chemistry Lett. 1980, 1415). Zunächst wird mit einem Trialkylboran, vorzugsweise Triethylboran, in THF bei Raumtemperatur umgesetzt und dann bei -78 °C bis 0 °C mit Natriumborhydrid, gegebenenfalls unter Zusatz von Methanol, reduziert. Auf diese Weise erhält man die in den vorhergehenden Ausführungen beschriebenen stereochemischen Verhältnisse.

Die racemischen Verbindungen der Formeln I und III können nach den bekannten Verfahren der Racematspaltung in die reinen Enantiomeren getrennt werden. Die Salze und Säuren der Verbindungen der allgemeinen Formel I werden nach den allgemein bekannten Methoden erhalten.

Das beschriebene Verfahren beinhaltet die Herstellung von Endprodukten und Zwischenprodukten mit X-Y gleich $CH_2$-$CH_2$ und HC=CH. Die Hydrierung der gegebenenfalls vorhandenen Doppelbindung kann gegebenenfalls entweder an Zwischenprodukten oder an den Endprodukten nach üblichen Verfahren vorgenommen werden.

Verbindungen der Formel I, für die gilt X-Y gleich $CH_2$-$CH_2$ werden z.B. durch katalytische Hydrierung aus Verbindungen der Formel I mit X-Y gleich CH=CH gewonnen. Die Hydrierung wird in Lösungsmitteln wie Methanol, Ethanol oder Essigester mit Katalysatoren wie Pd/C bei Normaldruck oder erhöhtem Druck ausgeführt.

Die Laktone der Formel II werden ebenfalls nach an sich bekannten Verfahren erhalten, beispielsweise durch Wasserabspaltung aus den offenen Dihydroxycarbonsäuren der Formel I, $R^4$ gleich H, in Benzol, Hexan oder Toluol unter Zusatz von p-Toluolsulfonsäure bei Raumtemperatur bis Rückflußtemperatur oder aber aus den offenen Dihydroxycarbonsäureestern der Formel I, z.B. $R^4$ = Methyl oder Ethyl in Dichlormethan unter Zusatz von z.B. Trifluoressigsäure bei Raumtemperatur bis Rückflußtemperatur.

Die Aldehyde der Formel III werden z.B. aus den Nitrilen der Formel VI

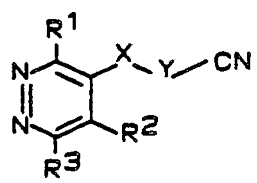

VI

gewonnen. Die Reaktion der Nitrile der Formel VI, worin X-Y, $R^1$, $R^2$ und $R^3$ die zu Formel I angegebene Bedeutung haben, mit Diisobutylaluminiumhydrid (DIBAH) in THF bei -10 °C bis 50 °C führt nach Hydrolyse direkt zu den Aldehyden der Formel III.

Die Nitrile der Formel VI werden aus den Aldehyden der Formel VII gewonnen,

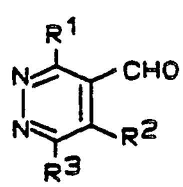

VII

worin $R^1$, $R^2$ und $R^3$ die angegebene Bedeutung haben, und zwar vorzugsweise durch Umsetzung mit Cyanomethanphosphonaten, insbesondere Cyanomethanphosphonsäurediisopropylester in Lösungsmitteln wie THF in Gegenwart einer Base, vorzugsweise Natriumhydrid bei Temperaturen zwischen -20 °C und Raumtemperatur. Es fallen überwiegend die E-Isomeren an.

Die Aldehyde der Formel VII werden durch Oxidation der Alkohole der Formel VIII gewonnen,

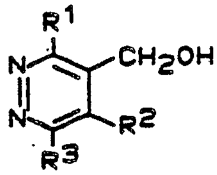

VIII

worin $R^1$, $R^2$ und $R^3$ die angegebene Bedeutung haben. Vorzugsweise wird die Oxidation mit Pyridiniumchlorochromat (PCC) in Dichlormethan bei Raumtemperatur ausgeführt.

Die Alkohole der Formel VIII entstehen durch Reduktion aus den Estern der Formel IX,

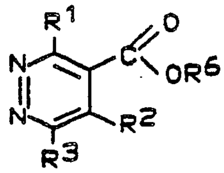

IX

worin $R^1$, $R^2$ und $R^3$ die angegebenen Bedeutungen haben. $R^6$ bedeutet eine geeignete Säureschutzgruppe, vorzugsweise Alkyl mit 1 bis 5 C-Atomen. Die Reduktion zu Verbindungen der Formel VIII wird vorzugsweise mit DIBAH in Dichlormethan oder Lithiumaluminiumhydrid in Ether oder THF bei -40 °C bis Raumtemperatur ausgeführt.

Die im erfindungsgemäßen Verfahren als Ausgangsmaterial verwandten Pyridazincarbonsäureester der allgemeinen Formel IX, worin $R^1$, $R^2$ und $R^3$ die zur allgemeinen Formel I gegebene Bedeutung haben, erhält man z.B. wie in Schema 1 dargestellt. Die Herstellung erfolgt nach in der Literatur beschriebenen Methoden oder analog solchen Methoden.

**Schema 1**

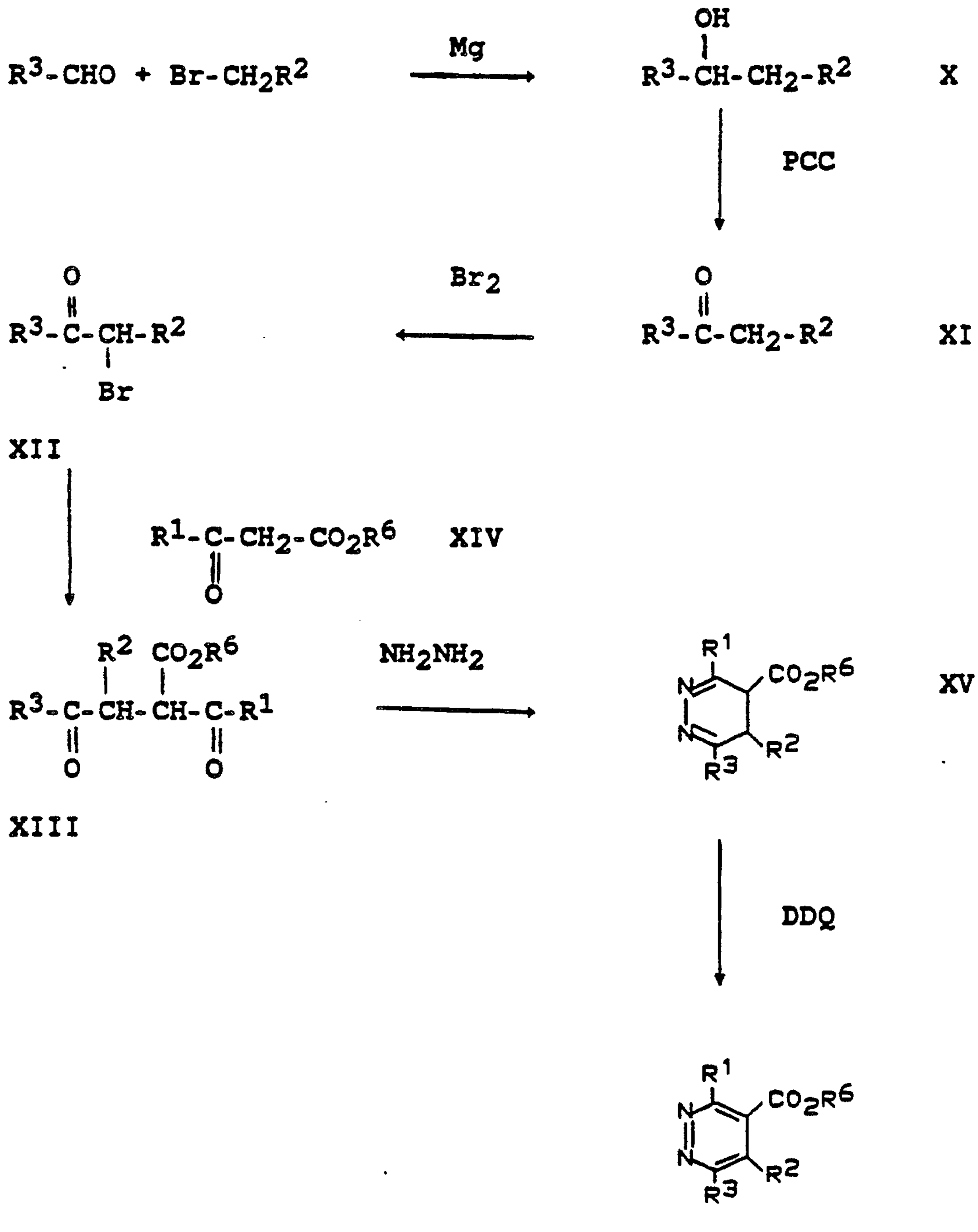


X

XI

XII

XIV

XIII

XV

IX

Analog der in Houben-Weyl, Methoden der organischen Chemie, Band 6/1a/2, S. 928 (1980) beschriebenen Methode führt z.B. die Umsetzung eines Grignard-Reagenzes $R^2CH_2MgHal$ mit einem Aldehyd $R^3$-CHO, wobei $R^2$ und $R^3$ die angegebene Bedeutung haben und Hal gleich Chlor, Brom und Jod, vorzugsweise Chlor bedeutet, zu Verbindungen der Formel X.

Die Alkohole der Formel X, wobei $R^2$ und $R^3$ die angegebene Bedeutung haben, werden nach literaturbekannten Verfahren zu den Ketonen der allgemeinen Formel XI oxidiert, vorzugsweise mit Pyridiniumchlorochromat (PCC) in Dichlormethan bei Raumtemperatur (E.J. Corey, Tetrahedron Lett. 1975, 2647).

Die Ketone der Formel XI setzt man z.B. mit Halogenen, wie Chlor, Brom oder Jod, in organischen Lösungsmitteln, wie Tetrachlorkohlenstoff, Chloroform oder Dichlormethan, bei Temperaturen zwischen 0 °C und 40 °C zu den Halogenketonen der allgemeinen Formel XII um, wobei $R^2$ und $R^3$ die angegebene Bedeutung haben. Die Umsetzung erfolgt vorzugsweise mit Brom in Dichlormethan bei Raumtemperatur.

Die Überführung der Halogenketone der allgemeinen Formel XII in 1,4-Diketone der allgemeinen Formel XIII, wobei $R^1$, $R^2$, $R^3$ und $R^6$ die angegebene Bedeutung haben, gelingt beispielsweise durch Umsetzung von β-Ketoestern der allgemeinen Formel XIV, wobei $R^1$ und $R^6$ die angegebene Bedeutung haben, mit den Halogenketonen der allgemeinen Formel XII.

Die Darstellung der β-Ketoester der allgemeinen Formel XIV erfolgt nach literaturbekannten Methoden (z.B. analog M. Jackman, M. Klenk, B. Fishburn, B.F. Tullar und S. Archer, J. Am. Chem. Soc. 70, 2884 (1948). Daher erhält man Verbindungen, worin $R^1$ die oben angegebene Bedeutung hat und $R^6$ einen geradkettigen oder verzweigten Alkylrest mit bis zu 6 C-Atomen bedeutet, vorzugsweise einen Methyl- oder Ethylrest.

Die Umsetzung zum 1,4-Diketon der allgemeinen Formel XIII gelingt z.B. durch die Salzbildung des β-Ketoesters der allgemeinen Formel XIV mittels Basen wie z.B. Natriumethylat oder Natriumhydrid (in situ) und anschließender Reaktion mit dem Halogenketon der allgemeinen Formel XII bei Temperaturen zwischen 0° C und 40° C, bevorzugt 20° C in einem Lösungsmittel wie z.B. Diethylether, 1,2-Dimethoxyethan, 1,2-Diethoxyethan, Tetrahydrofuran, oder Gemischen dieser Lösungsmittel, bevorzugt Tetrahydrofuran.

Die Darstellung von Verbindungen der Formel XV, worin $R^1$, $R^2$, $R^3$ und $R^6$ die angegebene Bedeutung haben, erfolgt vorzugsweise durch die Umsetzung des 1,4-Diketons der allgemeinen Formel XIII mit Hydrazin oder Hydrazinhydrat in einem protischen Lösungsmittel wie Methanol oder Ethanol, bevorzugt Ethanol bei Temperaturen zwischen 0° C und Siedetemperatur des Lösungsmittels nach literaturbekannten Verfahren (T.L. Jacobs, Heterocyclic Compounds (Hrsg. R.C. Elderfield), Vol. 6, New York; J. Wiley & Sons 1957, S. 101 und dort zitierte Literatur).

Die Dehydrierung zu Verbindungen der allgemeinen Formel IX, worin $R^1$, $R^2$, $R^3$ und $R^6$ die angegebene Bedeutung haben, gelingt vorzugsweise mit Dehydrierungsmitteln wie z.B. 2,3-Dichlor-5,6-dicyano-1,4-benzochinon oder Chloranil wie von E.A. Braude, J. Hannah, R. Linstead, J. Chem. Soc. 1960, 3257 beschrieben.

Die Reinigung von Zwischenprodukten erfolgt falls notwendig, durch Destillation, Kristallisation, Flash-Chromatographie oder HPLC.

Biologische Testsysteme:

1. HMG-CoA-Reduktase-Aktivität in Enzympräparationen

Die HMG-CoA-Reduktase-Aktivität wurde an solubilisierten Enzympräparationen aus Lebermikrosomen von Ratten gemessen, die nach Umstellung im Tag-Nacht-Rhythmus mit Cholestyramin (®Cuemid) induziert wurden. Als Substrat diente (S,R)$^{14}$C-HMG-CoA, die Konzentration von NADPH wurde während der Inkubation durch ein regenerierendes System aufrechterhalten. Die Abtrennung von $^{14}$C-Mevalonat vom Substrat und anderen Produkten (z.B. $^{14}$C-HMG) erfolgte über Säulenelution, wobei das Elutionsprofil jeder Einzelprobe ermittelt wurde. Auf die ständige Mitführung von $^{3}$H-Mevalonat wurde verzichtet, weil es sich bei der Bestimmung um die Relativangabe der Hemmwirkung handelt. In einer Versuchsreihe wurden jeweils die enzymfreie Kontrolle, der enzymhaltige Normalansatz (= 100 %) und solche mit Präparatezusätzen, Endkonzentration $10^{-5}$ bis $10^{-9}$ M, zusammen behandelt. Jeder Einzelwert wurde als Mittelwert aus 3 Parallelproben gebildet. Die Signifikanz der Mittelwertsunterschiede zwischen präparatefreien und präparatehaltigen Proben wurde nach dem t-Test beurteilt.

Nach der oben beschriebenen Methode wurden von den erfindungsgemäßen Verbindungen Hemmwerte auf die HMG-CoA-Reduktase ermittelt ($IC_{50}$/mol/Liter bedeutet die molare Konzentration der Verbindung pro Liter, die für eine 50 %ige Hemmung erforderlich ist):

Für die Beispiel 13 b bis k wurden $IC_{50}$-Werte zwischen $1 \bullet 10^{-9}$ und $1 \bullet 10^{-8}$ gefunden, speziell für Beispiel 13 a, $1{,}7 \bullet 10^{-9}$, die Verbindung liegt in racemischer Form vor. Der $IC_{50}$-Wert der Verbindung aus Beispiel 19 a liegt bei $1{,}3 \bullet 10^{-9}$.

2. Supprimierung bzw. Inhibierung der HMG-CoA-Reduktase in Zellkulturen von HEP-G2-Zellen.

Monolayers von HEP-G2-Zellen in lipoproteinfreiem Nährmedium wurden mit entsprechenden Konzentrationen der Prüfsubstanzen eine bestimmte Zeit (z.B. 1 Stunde) vorinkubiert, nach Zugabe des markierten Präkursors, z.B. $^{14}$C-Natriumacetat, wurde die Inkubation fortgesetzt (z.B. 3 Stunden). Nach Zugabe eines internen Standards ($^{3}$H-Cholesterin) wurde ein Teil der Zellen alkalisch verseift. Die Lipide der verseiften

Zellen wurden mit Chloroform/Methanol extrahiert. Dieses Lipidgemisch wurde nach Zusatz von Träger-Cholesterin präparativ dünnschichtchromatographisch getrennt, die Cholesterinbande nach dem Sichtbarmachen mit Jod-Dampf isoliert und die aus dem $^{14}C$-Präkursor gebildete Menge $^{14}C$-Cholesterin szintigraphisch bestimmt. In einem aliquoten Teil der Zellen wurde Zellprotein bestimmt, so daß die in der Zeiteinheit pro mg Zellprotein gebildete Menge $^{14}C$-Cholesterin berechnet werden kann. Durch Vergleich dieses Wertes mit der Menge an $^{14}C$-Cholesterin, die pro mg Zellprotein und Zeiteinheit in einer gleichbehandelten, jedoch prüfsubstanzfreien Kultur gebildet wurde, ergab sich die Hemmwirkung des jeweiligen Prüfpräparates auf die Cholesterin-Biosynthese von HEP-G2-Zellkulturen.

Prüfung der Substanzen auf Hemmung der Cholesterin-Biosynthese in Zellkulturen

| konfluente Zellkulture (Monolayer) von HEP-G2-Zellen | | |
|---|---|---|
| 1. | lipoproteinfreies Medium (DMEM) | 24 h |
| 2. | Inkubation mit Prüfpräparaten | 1 h |
| 3. | Inkubation mit $^{14}C$-Acetat | 3 h |
| 4. | Cytolyse | |
| 5. | DC-Trennung des Reaktionsprodukts $^{14}C$-Cholesterin | |
| 6. | Isolierung des $^{14}C$-Cholesterin | |
| 7. | Szintillationsmessung | |
| 8. | Ergebnis in nmol $^{14}C$-Cholesterin/mg Zellprotein im Vergleich zur Lösungsmittelkontrolle | |

Nach der oben beschriebenen Methode wurde von den erfindungsgemäßen Verbindungen Hemmwerte der Cholesterinbiosynthese (in HEP-G2-Zellen) ermittelt (der $IC_{50}$/mol ist diejenige Konzentration der Verbindung, die eine 50 %ige Hemmung der Cholesterinbiosynthese bewirkt).

Diese Hemmwerte liegen für die Verbindungen Beispiel 13 b bis k bei Werten zwischen $10^{-7}$ und $10^{-9}$ mol pro Liter, speziell für Beispiel 13 a bei $5 \cdot 10^{-8}$ mol pro Liter, für Beispiel 19 a bei $2,5 \cdot 10^{-8}$ mol pro Liter.

Die Verbindungen der allgemeinen Formel I bzw. II zeichen sich aus durch starke Hemmung der HMG-CoA-Reduktase, des geschwindigkeitsbestimmenden Enzyms der Cholesterinbiosynthese.

Das durch $IC_{50}$-Werte im Bereich von $10^{-7}$ bis $10^{-9}$ mol pro Liter charakterisierte Ausmaß der Hemmung ist bei Verbindungen der allgemeinen Formel I bzw. II deutlich höher als bei literaturbekannten, vollsynthetischen HMG-CoA-Reduktase-Inhibitoren wie z.B. den von G.E. Stokker et al., J. Med. Chem. 29, 170 (1986) beschriebenen.

Das Enzym HMG-CoA-Reduktase ist in der Natur weit verbreitet. Es katalysiert die Bildung von Mevalonsäure aus HMG-CoA. Diese Reaktion ist ein zentraler Schritt der Cholesterinbiosynthese (vgl. J.R. Sabine in CRC Series in Enzyme Biology: 3-Hydroxy-3-methylglutaryl Coenzym A Reductase, CRC Press Inc. Boca Raten, Florida 1983 (ISBN 0-8493-6551-1)).

Hohe Cholesterinspiegel werden mit einer Reihe von Erkrankungen, wie z.B. koronarer Herzkrankheit oder Arteriosklerose in Verbindung gebracht. Daher ist die Senkung erhöhter Cholesterinspiegel zur Vorbeugung und Behandlung solcher Erkrankungen ein therapeutisches Ziel.

Ein Ansatzpunkt dazu liegt in der Hemmung bzw. Verminderung der endogenen Cholesterinbiosynthese. Hemmstoffe der HMG-CoA-Reduktase blockieren die Cholesterinbiosynthese auf einer frühen Stufe.

Die Verbindungen der allgemeinen Formel I bzw. II eignen sich daher als Hypolipidemika und zur Behandlung bzw. Prophylaxe arteriosklerotischer Veränderungen.

Die Erfindung betrifft daher auch pharmazeutische Präparate auf Basis dieser Verbindungen sowie ihre Verwendung als Arzneimittel, insbesondere als Hypolipidemika und zur Prophylaxe arteriosklerotischer Veränderungen.

Die Anwendung der Verbindungen der Formel I bzw. II als Hypolipidemika oder Antiarteriosklerotika erfolgt in oralen Dosen von 3 bis 2500 mg, vorzugsweise jedoch im Dosisbereich von 10 bis 500 mg. Diese Tagesdosen können nach Bedarf auch in zwei bis vier Einzeldosen aufgeteilt werden oder in Retardform

verabreicht werden. Das Dosierungsschema kann vom Typ, Alter, Gewicht, Geschlecht und medizinischem Zustand des Patienten abhängen.

Ein zusätzlicher cholesterinsenkender Effekt läßt sich durch gleichzeitige Gabe der erfindungsgemäßen Verbindungen mit gallensäurebindenen Stoffen, wie z.B. Anionenaustauscherharzen, erzielen. Die Gallensäureausscheidung führt zu einer verstärkten Neusynthese und damit zu einem erhöhten Cholesterinabbau (vgl. M.S. Brown, P.T. Koranen und J.C. Goldstein, Science 212, 628 (1981); M.S. Brown, J.C. Goldstein, Spektrum der Wissenschaft 1985, 1, 96).

Die erfindungsgemäßen Verbindungen der Formel I bzw. II können in Form der δ-Lactone, als freie Säuren oder in Form ihrer physiologisch unbedenklichen anorganischen oder organischen Salze oder als Ester zur Anwendung kommen. Säuren und Salze bzw. Ester können in Form ihrer wäßrigen Lösungen oder Suspensionen oder auch gelöst oder suspendiert in pharmakologisch unbedenklichen organischen Lösungsmitteln wie ein- oder mehrwertigen Alkoholen wie z.B. Ethanol, Ethylenglykol oder Glycerin, in Triacetin, in Alkohol-Acetaldehyddiacetalgemischen, Ölen wie z.B. Sonnenblumenöl oder Lebertran, Ethern, wie z.B. Diethylenglykoldimethylether, oder auch Polyethern, wie z.B. Polyethylenglykol, oder auch in Gegenwart anderer pharmakologisch unbedenklicher Polymerträger wie z.B. Polyvinylpyrrolidon oder in festen Zubereitungen zur Anwendung gelangen.

Für die Verbindungen der Formel I bzw. II sind feste, oral verabreichbare Zubereitungsformen bevorzugt, die die üblichen Hilfsstoffe enthalten können. Sie werden nach üblichen Methoden hergestellt.

Als Zubereitungen für die orale Anwendung eignen sich insbesondere Tabletten, Dragees oder Kapseln. Eine Dosierungseinheit enthält vorzugsweise 10 bis 500 mg Wirkstoff.

Die Verbindungen der Formeln III, IV, V, VI, VII, VIII und IX sind neu und stellen wertvolle Zwischenprodukte für die Herstellung von Verbindungen der Formel I dar. Die Erfindung betrifft daher auch diese Verbindungen sowie Verfahren zu ihrer Herstellung.

Vorbemerkung: NMR-Spektren wurden, sofern nichts anderes angegeben, in $CDCl_3$ mit internem Standard TMS gemessen. Zur Klassifizierung von NMR-Signalen gelten folgende Abkürzungen: s = Singulett, d = Dublett, dd = Doppeldublett, tr = Triplett, q = Quartett, h = Heptett, m = Multiplett, br = breit.

Schmelzpunkte sind unkorrigiert.

Es gelten folgende Substituentenabkürzungen: i = iso, t = tertiär, c = cyclo.

**Beispiel 1**

Allgemeine Vorschrift zur Herstellung der Verbindungen der allgemeinen Formel X

Beispiel 1 a ($R^3$ = $R^2$ = 4-Fluorphenyl) 1,2-Di-(4-fluorphenyl)-ethanol

2,04 g (0,08 Mol) Magnesium werden unter Stickstoffatmosphäre vorgelegt und dann mit Ether überschichtet. Dazu tropft man 12,2 g (0,08 Mol) 4-Fluorbenzylchlorid in 20 ml Ether so zu, daß der Ether leicht siedet. Nach beendeter Zugabe wird weitere 30 Minuten zum Rückfluß erhitzt, auf Raumtemperatur abgekühlt und 8,3 g (0,07 Mol) 4-Fluorbenzaldehyd in 20 ml Ether langsam zugetropft. Falls die Lösung siedet, muß eventuell mit Eiswasser gekühlt werden. Nach beendeter Zugabe läßt man 2 Stunden rückflussieren, dann kühlt man ab und gießt auf 50 ml Eiswasser. Mit halbkonzentrierter Salzsäure wird angesäuert, die Phasen werden getrennt und die wäßrige Phase wird 5 x mit Ether extrahiert. Die vereinigten org. Phasen werden mit gesättigter Natriumhydrogencarbonatlösung neutralisiert, mit gesättigter Natriumchloridlösung gewaschen, über Magnesiumsulfat getrocknet und eingedampft. Das farblose Öl kristallisiert durch und ergibt 15,7 g weiße Kristalle.

Fp.: 48-50 °C

MS, $C_{14}H_{12}F_2O$ = 234 ($M^+$)

$^1$H-NMR: δ/ppm = 2,87 (d,2H); 2,20 (s,br, 1H); 4,70 (tr,1H); 6,8-7,3 (m,8H)

Analog zu Beispiel 1 a wurden die Beispiele der Tabelle 1 hergestellt:

$$R^3-\overset{OH}{\overset{|}{C}H}-CH_2-R^2$$

| Beispiel | $R^2$ | $R^3$ |
|---|---|---|
| 1 b | $4-FC_6H_4$ | $C_6H_5$ |
| 1 c | $C_6H_5$ | $C_6H_5$ |
| 1 d | $CH_3$ | $C_6H_5$ |
| 1 e | $4-FC_6H_4$ | $i-C_3H_7$ |
| 1 f | $4-FC_6H_4$ | $c-C_6H_{11}$ |
| 1 g | $i-C_3H_7$ | $C_6H_5$ |
| 1 h | $i-C_3H_7$ | $i-C_3H_7$ |
| 1 i | $4-FC_6H_4$ | $C_6H_5$ |
| 1 k | $4-C_6H_4CH_3$ | $4-C_6H_4CH_3$ |
| 1 l | $4-C_6H_4OCH_3$ | $4-C_6H_4OCH_3$ |
| 1 m | $4-FC_6H_4$ | $4-FC_6H_4$ |

**Beispiel 2**

Allgemeine Vorschrift zur Herstellung der Verbindungen der allgemeinen Formel XI

Beispiel 2 a ($R^3$ = $R^2$ = 4-Fluorphenyl) 4-Fluorbenzyl-4-fluorphenylketon

Unter Schutzgasatmosphäre wird 7,8 g (36 mmol) Pyridiniumchlorochromat in 80 ml Dichlormethan suspendiert. Dazu gibt man 8,5 g (36 mMol) der Verbindung aus Beispiel 1 a, gelöst in 20 ml Dichlormethan, langsam bei Raumtemperatur zu. Es wird 3 Stunden bei Raumtemperatur nachgerührt, das Reaktionsgemisch daraufhin über eine Kieselgelsäule filtriert (Lösungsmittel Ether) und eingedampft. Nach Umkristallisieren aus Methanol entstehen 6,4 g weiße Kristalle
Fp.: 91-93 °C
MS, $C_{14}H_{10}F_2O$ = 232 ($M^+$)
$^1$H-NMR: δ/ppm = 4,27 (s,2H); 6,87-7,47 (m, 6H); 7,90-8,23 (m,2H)

Analog zu Beispiel 2 a wurden die Beispiele der Tabelle hergestellt:

**Tabelle 2:**

$$R^3-\overset{\overset{O}{\|}}{C}-CH_2-R^2$$

| Beispiel | $R^2$ | $R^3$ | Molmasse |
|---|---|---|---|
| 2 b | $4-FC_6H_4$ | $C_6H_5$ | $C_{14}H_{11}FO$ 214[$M^+$] |
| 2 c | $C_6H_5$ | $C_6H_5$ | $C_{14}H_{12}O$ 196[$M^+$] |
| 2 d | $CH_3$ | $C_6H_5$ | $C_9H_{10}O$ 134[$M^+$] |
| 2 e | $4-FC_6H_4$ | $i-C_3H_7$ | $C_{11}H_{13}FO$ 180[$M^+$] |
| 2 f | $4-FC_6H_4$ | $c-C_6H_{11}$ | $C_{14}H_{17}FO$ 220[$M^+$] |
| 2 g | $i-C_3H_7$ | $C_6H_5$ | $C_{11}H_{14}O$ 162[$M^+$] |
| 2 h | $i-C_3H_7$ | $i-C_3H_7$ | $C_8H_{16}O$ 128[$M^+$] |
| 2 i | $4-FC_6H_4$ | $C_6H_5$ | $C_{14}H_{11}FO$ 214[$M^+$] |
| 2 k | $4-C_6H_4CH_3$ | $4-C_6H_4CH_3$ | $C_{16}H_{16}O$ 224[$M^+$] |
| 2 l | $4-C_6H_4OCH_3$ | $4-C_6H_4OCH_3$ | $C_{16}H_{16}O_3$ 256 [$M^+$] |
| 2 m | $4-FC_6H_4$ | $4-FC_6H_4$ | $C_{14}H_{10}F_2O$ 232 [$M^+$] |

**Beispiel 3**

Allgemeine Vorschrift zur Herstellung der Verbindungen der allgemeinen Formel XII

Beispiel 3 a ($R^3$ = $R^2$ = 4-Fluorphenyl) α-Brom-α-(4-fluorphenyl)-4-fluoracetophenon

Bei Raumtemperatur wird 1,5 g (6,5 mmol) der Verbindung gemäß Beispiel 2 a in 20 ml $CHCl_3$ gelöst und 1/10 von 3,34 ml (6,5 mmol) Brom zugegeben (es entsteht dunkelorange Farbe). Die entstandene Lösung wird vorsichtig erhitzt, bis die Lösung sich gelb färbt und dann auf 0° C abgekühlt. Das restliche Brom wird zugegeben und das Reaktionsgemisch für 16 Stunden bei Raumtemperatur gerührt. Die orange

Lösung wird 3 x mit gesättigter $NaHCO_3$-Lösung extrahiert, dann einmal mit gesättigter Natriumchloridlösung gewaschen. Nach Trocknen über Magnesiumsulfat entsteht nach Einengen ein oranges Öl, welches teilweise kristallisiert. Das Produkt (1,9 g) wird durch Kristallisation aus Ethanol als weißes Pulver erhalten.

Fp.: 75-78° C

MS, $C_{14}H_9BrF_2O$ = 310/312 ($M^+$)

$^1$H-NMR: δ/ppm = 6,9-8,0 (m,9H)

Analog zu Beispiel 3 a wurden die Beispiele der Tabelle 3 hergestellt.

**Tabelle 3:**

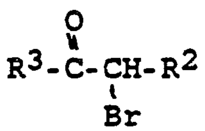

| Beispiel | $R^2$ | $R^3$ | Molmasse |
|---|---|---|---|
| 3 b | 4-$FC_6H_4$ | $C_6H_5$ | $C_{14}H_{10}BrFO$ 292/294[$M^+$] |
| 3 c | $C_6H_5$ | $C_6H_5$ | $C_{14}H_{12}BrO$ 274/276[$M^+$] |
| 3 d | $CH_3$ | $C_6H_5$ | $C_9H_9BrO$ 212/214[$M^+$] |

**Fortsetzung Tabelle 3**

| Beispiel | $R^2$ | $R^3$ | Molmasse |
|---|---|---|---|
| 3 e | 4-$FC_6H_4$ | i-$C_3H_7$ | $C_{11}H_{12}BrFO$ 258/260[$M^+$] |
| 3 f | 4-$FC_6H_4$ | c-$C_6H_{11}$ | $C_{14}H_{16}BrFO$ 298/300[$M^+$] |
| 3 g | i-$C_3H_7$ | $C_6H_5$ | $C_{11}H_{13}BrO$ 240/242[$M^+$] |
| 3 h | i-$C_3H_7$ | i-$C_3H_7$ | $C_8H_{15}BrO$ 206/208[$M^+$] |
| 3 i | 4-$FC_6H_4$ | $C_6H_5$ | $C_{16}H_{10}BrFO$ 292/294[$M^+$] |
| 3 k | 4-$C_6H_4CH_3$ | 4-$C_6H_4CH_3$ | $C_{14}H_{15}BrO$ 302/304[$M^+$] |
| 3 l | 4-$C_6H_4OCH_3$ | 4-$C_6H_4OCH_3$ | $C_{16}H_{15}BrO_3$ 334/336 [$M^+$] |
| 3 m | 4-$FC_6H_4$ | 4-$FC_6H_4$ | $C_{14}H_9BrF_2O$ 310/312 [$M^+$] |

**Beispiel 4**

Allgemeine Vorschrift zur Herstellung der Verbindungen der allgemeinen Formel XIII

Beispiel 4 a ($R^3$ = $R^2$ = 4-Fluorphenyl, $R^1$ = i-$C_3H_7$, $R^6$ = $C_2H_5$)

Ethyl-2-(α-4-fluorbenzoyl-4-fluorbenzyl)-3-oxo-4,4-dimethylpentanoat

Unter trockener Stickstoffatmosphäre werden 0,2 g (8 mMol) Natriumhydrid (60 % im Paraffinöl) in einem Kolben 2 x mit n-Heptan gewaschen und in 20 ml trockenem Tetrahydrofuran suspendiert. Dazu tropft man bei 0 °C 0,8 g (5,1 mMol) Isopropylacetessigsäureethylester (XIV) in 20 ml THF. Nach erfolgter Zugabe rührt man 15 Minuten bei 0 °C nach (die Lösung wird klar). Dann gibt man bei 0 °C 1,5 g (4,8 mMol) der Verbindung gemäß Beispiel 3 a, in 5 ml THF abs. gelöst, langsam zu und rührt 21 Stunden bei Raumtemperatur.

Das Reaktionsgemisch wird eingeengt, mit Ether aufgenommen, 2 x mit gesättigter Natriumhydrogencarbonat und 1 x mit gesättigter Natriumchloridlösung gewaschen, über Magnesiumsulfat getrocknet und eingedampft. Das entstandene gelbe Öl (1,4 g) wird direkt in die nächste Stufe (Beispiel 5 a) eingesetzt.

MS, $C_{22}H_{22}F_2O_4$ = 389 $(M+H^+)$

Analog zu Beispiel 4 a wurden die Beispiele der Tabelle 4 hergestellt unter Verwendung der entsprechenden $R^1$-substituierten Acetessigsäureethylester XIV

**Tabelle 4:**

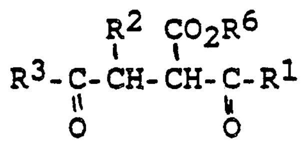

| Beispiel | $R^1$ | $R^2$ | $R^3$ | $R^6$ |
|---|---|---|---|---|
| 4 b | $CH_3$ | 4-$FC_6H_4$ | $C_6H_5$ | $C_2H_5$ |
| 4 c | $CH_3$ | $C_6H_5$ | $C_6H_5$ | $C_2H_5$ |
| 4 d | i-$C_3H_7$ | $CH_3$ | $C_6H_5$ | $C_2H_5$ |
| 4 e | i-$C_3H_7$ | 4-$FC_6H_4$ | i-$C_3H_7$ | $C_2H_5$ |
| 4 f | i-$C_3H_7$ | 4-$FC_6H_4$ | c-$C_6H_{11}$ | $C_2H_5$ |
| 4 g | 4-$FC_6H_4$ | i-$C_3H_7$ | $C_6H_5$ | $C_2H_5$ |
| 4 h | 4-$FC_6H_4$ | i-$C_3H_7$ | i-$C_3H_7$ | $C_2H_5$ |
| 4 i | t-$C_4H_9$ | 4-$FC_6H_4$ | $C_6H_5$ | $C_2H_5$ |
| 4 k | i-$C_3H_7$ | 4-$C_6H_4CH_3$ | 4-$C_6H_4CH_3$ | $C_2H_5$ |
| 4 l | i-$C_3H_7$ | 4-$C_6H_4OCH_3$ | 4-$C_6H_4OCH_3$ | $C_2H_5$ |
| 4 m | $CH_3$ | 4-$FC_6H_4$ | 4-$FC_6H_4$ | $C_2H_5$ |

**Beispiel 5:**

Allgemeine Vorschrift zur Herstellung der Verbindungen der allgemeinen Formel XV

Beispiel 5 a ($R^3$ = $R^2$ = 4-Fluorphenyl, $R^1$ = i-$C_3H_7$, $R^6$ = $C_2H_5$)

3,4-Di-(4-fluorphenyl)-6-isopropyl-4,5-dihydro-pyridazin-5-carbonsäureethylester

Unter Stickstoffatmosphäre werden 1,43 g (3,7 mMol) der Verbindung aus Beispiel 4 a in 10 ml Ethanol gelöst und auf 0 °C abgekühlt. Bei dieser Temperatur werden 0,2 ml (3,7 mMol) Hydrazin-Hydrat langsam zugetropft. Man läßt auf Raumtemperatur kommen und rührt 1 Stunde. Die Reaktionslösung wird eingedampft. Flash-Chromatographie auf Kieselgel (Cyclohexan/Ethylacetat 3/1) ergibt 0,31 g der Titelverbindung als farbloses Öl.

Die Verbindung der Formel XV wird direkt in die nächste Stufe ohne weitere Charakterisierung eingesetzt (Beispiel 6a). MS, $C_{22}H_{22}F_2N_2O_2$ = 385 (M + $H^+$)

Analog zu Beispiel 5 a wurden die Beispiele der Tabelle 5 hergestellt:

**Tabelle 5:**

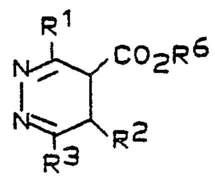

| Beispiel | $R^1$ | $R^2$ | $R^3$ | $R^6$ |
|---|---|---|---|---|
| 5 b | $CH_3$ | 4-$FC_6H_4$ | $C_6H_5$ | $C_2H_5$ |
| 5 c | $CH_3$ | $C_6H_5$ | $C_6H_5$ | $C_2H_5$ |
| 5 d | i-$C_3H_7$ | $CH_3$ | $C_6H_5$ | $C_2H_5$ |

**Fortsetzung von Tabelle 5**

| Beispiel | $R^1$ | $R^2$ | $R^3$ | $R^6$ |
|---|---|---|---|---|
| 5 e | i-$C_3H_7$ | 4-$FC_6H_4$ | i-$C_3H_7$ | $C_2H_5$ |
| 5 f | i-$C_3H_7$ | 4-$FC_6H_4$ | c-$C_6H_{11}$ | $C_2H_5$ |
| 5 g | 4-$FC_6H_4$ | i-$C_3H_7$ | $C_6H_5$ | $C_2H_5$ |
| 5 h | 4-$FC_6H_4$ | i-$C_3H_7$ | i-$C_3H_7$ | $C_2H_5$ |
| 5 i | t-$C_4H_9$ | 4-$FC_6H_4$ | $C_6H_5$ | $C_2H_5$ |
| 5 k | i-$C_3H_7$ | 4-$C_6H_4CH_3$ | 4-$C_6H_4CH_3$ | $C_2H_5$ |
| 5 l | i-$C_3H_7$ | 4-$C_6H_4OCH_3$ | 4-$C_6H_4OCH_3$ | $C_2H_5$ |
| 5 m | $CH_3$ | 4-$FC_6H_4$ | 4-$FC_6H_4$ | $C_2H_5$ |

**Beispiel 6:**

Allgemeine Vorschrift zur Herstellung der Verbindungen der allgemeinen Formel IX

Beispiel 6 a ($R^3$ = $R^2$ = 4-Fluorphenyl, $R^1$ = i-$C_3H_7$, $R^6$ = $C_2H_5$)

3,4-Di-(4-fluorphenyl)-6-isopropyl-pyridazin-5-carbonsäureethylester

Unter Stickstoffatmosphäre werden 260 mg (0,7 mMol) der Verbindung aus Beispiel 5 a und 170 mg (0,7 mMol) Dichlordicyanobenzochinon in 25 ml Toluol vorgelegt und 3 Stunden bei 50 °C gerührt.

Das Reaktionsgemisch wird eingeengt und über eine Kieselgelsäule filtriert (Cyclohexan/Ethylacetat 3/1). Das Produkt (166 mg) kristallisiert aus.

Fp.: 103 °C

MS, $C_{22}H_{20}F_2N_2O_2$ = 382 ($M^+$)

$^1$H-NMR: δ/ppm = 1,00 (tr,J=7Hz,3H); 1,50 (d,J=7Hz,6H); 3,23 (h,1H), 4,13 (q,J=7Hz,2H); 6,90-7,53 (m,8H)

In Analogie zu Beispiel 6 a wurden die Beispiele der Tabelle 6 erhalten.

**Tabelle 6:**

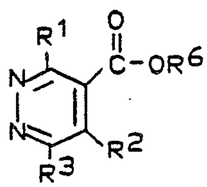

| Beispiel | $R^1$ | $R^2$ | $R^3$ | $R^6$ | Molmasse |
|---|---|---|---|---|---|
| 6 b | $CH_3$ | $4\text{-}FC_6H_4$ | $C_6H_5$ | $C_2H_5$ | $C_{20}H_{17}FN_2O_2$ 336[$M^+$] |
| 6 c | $CH_3$ | $C_6H_5$ | $C_6H_5$ | $C_2H_5$ | $C_{20}H_{18}N_2O_2$ 318[$M^+$] |
| 6 d | $i\text{-}C_3H_7$ | $CH_3$ | $C_6H_5$ | $C_2H_5$ | $C_{17}H_{20}N_2O_2$ 284[$M^+$] |
| 6 e | $i\text{-}C_3H_7$ | $4\text{-}FC_6H_4$ | $i\text{-}C_3H_7$ | $C_2H_5$ | $C_{19}H_{23}FN_2O_2$ 330[$M^+$] |
| 6 f | $i\text{-}C_3H_7$ | $4\text{-}FC_6H_4$ | $c\text{-}C_6H_{11}$ | $C_2H_5$ | $C_{22}H_{27}FN_2O_2$ 370[$M^+$] |
| 6 g | $4\text{-}FC_6H_4$ | $i\text{-}C_3H_7$ | $C_6H_5$ | $C_2H_5$ | $C_{22}H_{21}FN_2O_2$ 364[$M^+$] |
| 6 h | $4\text{-}FC_6H_4$ | $i\text{-}C_3H_7$ | $i\text{-}C_3H_7$ | $C_2H_5$ | $C_{19}H_{23}FN_2O_2$ 330[$M^+$] |
| 6 i | $t\text{-}C_4H_9$ | $4\text{-}FC_6H_4$ | $C_6H_5$ | $C_2H_5$ | $C_{23}H_{23}FN_2O_2$ 378[$M^+$] |
| 6 k | $i\text{-}C_3H_7$ | $4\text{-}C_6H_4CH_3$ | $4\text{-}C_6H_4CH_3$ | $C_2H_5$ | $C_{24}H_{26}N_2O_2$ 374[$M^+$] |
| 6 1 | $i\text{-}C_3H_7$ | $4\text{-}C_6H_4OCH_3$ | $4\text{-}C_6H_4OCH_3$ | $C_2H_5$ | $C_{24}H_{26}N_2O_4$ 406 [$M^+$] |
| 6 m | $CH_3$ | $4\text{-}FC_6H_4$ | $4\text{-}FC_6H_4$ | $C_2H_5$ | $C_{20}H_{16}F_2N_2O_2$ 354 [$M^+$] |

**Beispiel 7:**

Allgemeine Vorschrift zur Darstellung der Verbindungen der allgemeinen Formel VIII

Beispiel 7 a ($R^1$ = i-$C_3H_7$, $R^2$ = 4-$FC_6H_4$, $R^3$ = 4-$FC_6H_4$)

3,4-Di-(4-fluorphenyl)-6-isopropyl-pyridazin-5-yl-methanol

Zu 2,4 g (6,2 mMol) der Verbindung gemäß Beispiel 6 a in 230 ml Dichlormethan werden bei -78 °C unter Argon 15,4 ml (18,5 mmol) Diisobutylaluminiumhydridlösung (Toluol) zugetropft. Man rührt 4 h bei 0 °C, gibt 1 ml Methanol zu und verdünnt mit Wasser und Ether bis zur Phasentrennung. Die organische Phase wird abgetrennt, die wäßrige Phase nochmals mit Ether extrahiert und die vereinigten organischen Extrakte mit gesättigter Natriumchloridlösung (2 x) gewaschen. Nach Trocknen mit Magnesiumsulfat wird das Lösungsmittel im Vakuum abgezogen. Der entstandene Alkohol 7 a (1,9 g) wird ohne weitere Reinigung zur nächsten Stufe eingesetzt.

Öl

MS, $C_{20}H_{18}F_2N_2O$ = 340 ($M^+$)

$^1$H-NMR: δ/ppm = 1,50 (d,J=7Hz,6H); 3,63 (h,1H); 4,60 (s, 2H); 6,73-7,43 (m,8H)

In Analogie zu Beispiel 7 a wurden die Beispiele der Tabelle 7 erhalten.

**Tabelle 7:**

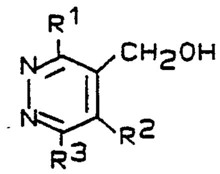

| Beispiel | $R^1$ | $R^2$ | $R^3$ | Molmasse |
|---|---|---|---|---|
| 7 b | $CH_3$ | 4-$FC_6H_4$ | $C_6H_5$ | $C_{18}H_{15}FN_2O$ 294[$M^+$] |
| 7 c | $CH_3$ | $C_6H_5$ | $C_6H_5$ | $C_{18}H_{10}N_2O$ 276[$M^+$] |
| 7 d | i-$C_3H_7$ | $CH_3$ | $C_6H_5$ | $C_{15}H_{18}N_2O$ 242[$M^+$] |

Fortsetzung Tabelle 7

| | | | | |
|---|---|---|---|---|
| 7 e | i-$C_3H_7$ | 4-$FC_6H_4$ | i-$C_3H_7$ | $C_{17}H_{21}FN_2O$ 288[$M^+$] |
| 7 f | i-$C_3H_7$ | 4-$FC_6H_4$ | c-$C_6H_{11}$ | $C_{20}H_{25}FN_2O$ 328[$M^+$] |
| 7 g | 4-$FC_6H_4$ | i-$C_3H_7$ | $C_6H_5$ | $C_{20}H_{19}FN_2O$ 322[$M^+$] |
| 7 h | 4-$FC_6H_4$ | i-$C_3H_7$ | i-$C_3H_7$ | $C_{17}H_{21}FN_2O$ 288[$M^+$] |
| 7 i | t-$C_4H_9$ | 4-$FC_6H_4$ | $C_6H_5$ | $C_{21}H_{21}FN_2O$ 336[$M^+$] |
| 7 k | i-$C_3H_7$ | 4-$C_6H_4CH_3$ | 4-$C_6H_4CH_3$ | $C_{22}H_{24}N_2O$ 332[$M^+$] |
| 7 l | i-$C_3H_7$ | 4-$C_6H_4OCH_3$ | 4-$C_6H_4OCH_3$ | $C_{22}H_{24}N_2O_4$ 364 [$M^+$] |
| 7 m | $CH_3$ | 4-$FC_6H_4$ | 4-$FC_6H_4$ | $C_{18}H_{14}F_2N_2O$ 312 [$M^+$] |

**Beispiel 8:**

Allgemeine Vorschrift zur Darstellung der Verbindungen der allgemeinen Formel VII

Beispiel 8 a ($R^1$ = i-$C_3H_7$, $R^2$ = 4-$FC_6H_4$, $R^3$ = 4-$FC_6H_4$)

3,4-Di-(4-fluorphenyl)-6-isopropyl-pyridazin-5-aldehyd

Zu einer Suspension von 1,8 g (8,4 mmol) PCC in 30 ml Dichlormethan werden bei Raumtemperatur 1,8 g (5,5 mmol) der Verbindung gemäß Beispiel 7 a in 30 ml Dichlormethan gegossen. Nach 3 h Rühren bei Raumtemperatur wird mit 3 Volumen Ether versetzt und über Kieselgel filtriert. Abziehen des Lösungsmittels im Vakuum und Chromatographie an Kieselgel (Cyclohexan/Ethylacetat 1/1) ergibt 1,5 g der Titelverbindung als Festkörper.
Fp.: 136 °C
MS, $C_{20}H_{16}F_2N_2O$ = 338 ($M^+$)
$^1$H-NMR: δ/ppm = 1,47 (d,J=7Hz,6H); 3,73 (h,1H); 6,83-7,50 (m,8H); 10,00 (s,1H)

In Analogie zu Beispiel 8 a wurden die Beispiele der Tabelle 8 erhalten.

**Tabelle 8:**

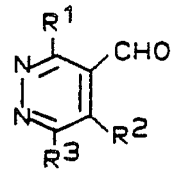

| Beispiel | $R^1$ | $R^2$ | $R^3$ | Molmasse |
|---|---|---|---|---|
| 8 b | $CH_3$ | $4\text{-}FC_6H_4$ | $C_6H_5$ | $C_{18}H_{13}FN_2O$ 292[$M^+$] |
| 8 c | $CH_3$ | $C_6H_5$ | $C_6H_5$ | $C_{18}H_{14}N_2O$ 274[$M^+$] |
| 8 d | $i\text{-}C_3H_7$ | $CH_3$ | $C_6H_5$ | $C_{15}H_{16}N_2O$ 240[$M^+$] |
| 8 e | $i\text{-}C_3H_7$ | $4\text{-}FC_6H_4$ | $i\text{-}C_3H_7$ | $C_{17}H_{19}FN_2O$ 286[$M^+$] |
| 8 f | $i\text{-}C_3H_7$ | $4\text{-}FC_6H_4$ | $c\text{-}C_6H_{11}$ | $C_{20}H_{23}FN_2O$ 326[$M^+$] |
| 8 g | $4\text{-}FC_6H_4$ | $i\text{-}C_3H_7$ | $C_6H_5$ | $C_{20}H_{17}FN_2O$ 320[$M^+$] |
| 8 h | $4\text{-}FC_6H_4$ | $i\text{-}C_3H_7$ | $i\text{-}C_3H_7$ | $C_{17}H_{19}FN_2O$ 286[$M^+$] |
| 8 i | $t\text{-}C_4H_9$ | $4\text{-}FC_6H_4$ | $C_6H_5$ | $C_{21}H_{19}FN_2O$ 334[$M^+$] |
| 8 k | $i\text{-}C_3H_7$ | $4\text{-}C_6H_4CH_3$ | $4\text{-}C_6H_4CH_3$ | $C_{22}H_{22}N_2O$ 330[$M^+$] |
| 8 l | $i\text{-}C_3H_7$ | $4\text{-}C_6H_4OCH_3$ | $4\text{-}C_6H_4OCH_3$ | $C_{22}H_{22}N_2O_3$ 362 [$M^+$] |
| 8 m | $CH_3$ | $4\text{-}FC_6H_4$ | $4\text{-}FC_6H_4$ | $C_{18}H_{12}F_2N_2O$ 310 [$M^+$] |

**Beispiel 9:**

Allgemeine Vorschrift zur Darstellung der Verbindungen der allgemeinen Formel VI

Beispiel 9 a ($R^1$ = $i\text{-}C_3H_7$, $R^2$ = $4\text{-}FC_6H_4$, $R^3$ = $4\text{-}FC_6H_4$, X-Y = CH=CH

3,4-Di-(4-fluorphenyl)-6-isopropyl-pyridazin-5-ethenyl-cyanid

242 mg (5,6 mmol) Natriumhydrid werden unter Argon vom Weißöl befreit und in 20 ml THF vorgelegt. Bei 0 °C werden 0,9 ml (4,6 mmol) Cyanomethanphosphonsäurediisopropylester zugespritzt. Nach beendeter Gasentwicklung (ca. 30 Minuten) werden 1,3 g (4 mmol) der Verbindung gemäß Beispiel 8 a in 20 ml THF zugetropft und 3 Stunden bei Raumtemperatur gerührt. Die Mischung wird auf gesättigte NaCl-Lösung gegossen und 3 x mit Ether extrahiert. Die vereinigten organischen Phasen werden einmal mit gesättigter NaCl-Lösung gewaschen und getrocknet ($MgSO_4$). Abziehen des Lösungsmittels im Vakuum ergibt nach Chromatographie auf Kieselgel (Cyclohexan/Ethylacetat 1/1) 985 mg eines weißen Festkörpers (Titelverbindung).

Fp.: 160 °C

MS, $C_{22}H_{17}F_2N_3$ = 361 ($M^+$)

$^1$H-NMR: δ/ppm = 1,50 (d,J=7Hz,6H); 3,40 (h,1H); 5,33 (d,J=16Hz,1H); 6,83-7,55 (m,8H)

In Analogie zu Beispiel 9 a wurden die Beispiele der Tabelle 9 erhalten.

**Tabelle 9:**

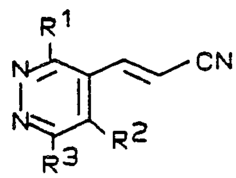

| Beispiel | $R^1$ | $R^2$ | $R^3$ | Molmasse |
|---|---|---|---|---|
| 9 b | $CH_3$ | $4\text{-}FC_6H_4$ | $C_6H_5$ | $C_{20}H_{14}FN_3$ 315[$M^+$] |
| 9 c | $CH_3$ | $C_6H_5$ | $C_6H_5$ | $C_{20}H_{15}N_3$ 297[$M^+$] |
| 9 d | $i\text{-}C_3H_7$ | $CH_3$ | $C_6H_5$ | $C_{17}H_{17}N_3$ 263[$M^+$] |
| 9 e | $i\text{-}C_3H_7$ | $4\text{-}FC_6H_4$ | $i\text{-}C_3H_7$ | $C_{19}H_{20}FN_3$ 309[$M^+$] |
| 9 f | $i\text{-}C_3H_7$ | $4\text{-}FC_6H_4$ | $c\text{-}C_6H_{11}$ | $C_{22}H_{24}FN_3$ 349[$M^+$] |
| 9 g | $4\text{-}FC_6H_4$ | $i\text{-}C_3H_7$ | $C_6H_5$ | $C_{22}H_{18}FN_3$ 343[$M^+$] |
| 9 h | $4\text{-}FC_6H_4$ | $i\text{-}C_3H_7$ | $i\text{-}C_3H_7$ | $C_{19}H_{20}FN_3$ 309[$M^+$] |
| 9 i | $t\text{-}C_4H_9$ | $4\text{-}FC_6H_4$ | $C_6H_5$ | $C_{23}H_{20}FN_3$ 357[$M^+$] |
| 9 k | $i\text{-}C_3H_7$ | $4\text{-}C_6H_4CH_3$ | $4\text{-}C_6H_4CH_3$ | $C_{24}H_{23}N_3$ 353[$M^+$] |
| 9 l | $i\text{-}C_3H_7$ | $4\text{-}C_6H_4OCH_3$ | $4\text{-}C_6H_4OCH_3$ | $C_{24}H_{23}N_3O_2$ 385 [$M^+$] |
| 9 m | $CH_3$ | $4\text{-}FC_6H_4$ | $4\text{-}FC_6H_4$ | $C_{20}H_{13}F_2N_3$ 357 [$M^+$] |

**Beispiel 10:**

Allgemeine Vorschrift zur Darstellung der Verbindungen der allgemeinen Formel III

Beispiel 10 a ($R^1$ = $i\text{-}C_3H_7$, $R^2$ = $4\text{-}FC_6H_4$, $R^3$ = $4\text{-}FC_6H_4$, X-Y = CH=CH

3,4-Di-(4-fluorphenyl)-6-isopropyl-pyridazin-5-propenal

Zu 975 mg (2,7 mmol) der Verbindung gemäß Beispiel 9 a in 20 ml THF werden bei 0° C 4,5 ml (5,4 mmol) Diisobutylaluminiumhydrid-Lösung (in Toluol) gespritzt. Nach 4 Stunden bei 10° C wird vorsichtig mit 1 N Salzsäure hydrolysiert und mit Ethylacetat extrahiert (3 x). Die vereinigten org. Phasen werden einmal mit gesättigter Natriumhydrogencarbonatlösung gewaschen und getrocknet ($MgSO_4$). Abziehen des Lösungsmittels im Vakuum und Flash-Chromatographie auf Kieselgel (Cyclohexan/Ethylacetat 1/1) ergibt 600 mg eines farblosen Öles (Titelverbindung).
MS, $C_{22}H_{18}F_2N_2O$ = 365 ($M^+$)
$^1$H-NMR: δ/ppm = 1,57 (d,J=7Hz,6H); 3,47 (h,1H); 6,20 (dd,$J_1$=16Hz,$J_2$=7Hz,1H); 6,77-7,60 (m,8H); 9,60 (d,J=7Hz,1H)

In Analogie zu Beispiel 10 a wurden die Beispiele der Tabelle 10 erhalten.

**Tabelle 10:**

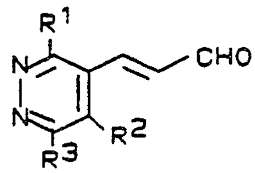

| Beispiel | $R^1$ | $R^2$ | $R^3$ | Molmasse |
|---|---|---|---|---|
| 10 b | $CH_3$ | $4\text{-}FC_6H_4$ | $C_6H_5$ | $C_{20}H_{15}FN_2O$ 318[$M^+$] |
| 10 c | $CH_3$ | $C_6H_5$ | $C_6H_5$ | $C_{20}H_{16}N_2O$ 300[$M^+$] |
| 10 d | $i\text{-}C_3H_7$ | $CH_3$ | $C_6H_5$ | $C_{17}H_{18}N_2O$ 266[$M^+$] |
| 10 e | $i\text{-}C_3H_7$ | $4\text{-}FC_6H_4$ | $i\text{-}C_3H_7$ | $C_{19}H_{21}FN_2O$ 312[$M^+$] |
| 10 f | $i\text{-}C_3H_7$ | $4\text{-}FC_6H_4$ | $c\text{-}C_6H_{11}$ | $C_{22}H_{25}FN_2O$ 352[$M^+$] |
| 10 g | $4\text{-}FC_6H_4$ | $i\text{-}C_3H_7$ | $C_6H_5$ | $C_{22}H_{19}FN_2O$ 346[$M^+$] |

Fortsetzung Tabelle 10

| Beispiel | $R^1$ | $R^2$ | $R^3$ | Molmasse |
|---|---|---|---|---|
| 10 h | $4\text{-}FC_6H_4$ | $i\text{-}C_3H_7$ | $i\text{-}C_3H_7$ | $C_{19}H_{21}FN_2O$ 312[$M^+$] |
| 10 i | $t\text{-}C_4H_9$ | $4\text{-}FC_6H_4$ | $C_6H_5$ | $C_{23}H_{21}FN_2O$ 360[$M^+$] |
| 10 k | $i\text{-}C_3H_7$ | $4\text{-}C_6H_4CH_3$ | $4\text{-}C_6H_4CH_3$ | $C_{24}H_{24}N_2O$ 356[$M^+$] |
| 10 l | $i\text{-}C_3H_7$ | $4\text{-}C_6H_4OCH_3$ | $4\text{-}C_6H_4OCH_3$ | $C_{24}H_{24}N_2O_3$ 388 [$M^+$] |
| 10 m | $CH_3$ | $4\text{-}FC_6H_4$ | $4\text{-}FC_6H_4$ | $C_{20}H_{14}F_2N_2O$ 336 [$M^+$] |

**Beispiel 11:**

Allgemeine Vorschrift zur Darstellung von Verbindungen der allgemeinen Formel IV

Beispiel 11 a ($R^1$ = i-$C_3H_7$, $R^2$ = 4-$FC_6H_4$, $R^3$ = 4-$FC_6H_4$, X-Y = CH=CH, $R^4$ = $C_2H_5$

3,4-Di-(4-fluorphenyl)-6-isopropyl-pyridazin-5-(5-hydroxy-3-oxo-6-heptensäureethylester)

96 mg (2,2 mMol) vom Weißöl befreites Natriumhydrid werden unter Argon in 3 ml trockenem THF vorgelegt. Bei 0° C spritzt man 265 µl (2,8 mMol) Acetessigsäureethylester zu und rührt 15 Minuten bei 0° C. Anschließend werden 1,3 ml (2,0 mMol) BuLi (Hexan) bei 0° C zugespritzt. Nach 15 Minuten Rühren werden bei -70° C 500 mg (1,4 mMol) der Verbindung gemäß Beispiel 10 a in 5 ml THF zugetropft und 2,5 Stunden bei -70° C gerührt. Man läßt auf 0° C kommen, hydrolysiert mit kalter 2 N Salzsäure und extrahiert 3 x mit Ether. Die vereinigten organischen Extrakte werden einmal mit gesättigter Natriumchlorid-Lösung gewaschen und getrocknet ($MgSO_4$). Abziehen des Lösungsmittels ergibt 650 mg, Beispiel 11 a.

Die Verbindung des Beispiels 11 a wird ohne weitere Reinigung und Charakterisierung zur nächsten Stufe eingesetzt.

Öl

MS, $C_{28}H_{28}F_2N_2O_4$ = 495 ($M^+$)

$^1$H-NMR: Verbindung wird ohne weitere Charakterisierung in Beispiel 12 a eingesetzt.

In Analogie zu Beispiel 11 a wurden die Beispiele der Tabelle 11 erhalten.

**Tabelle 11:**

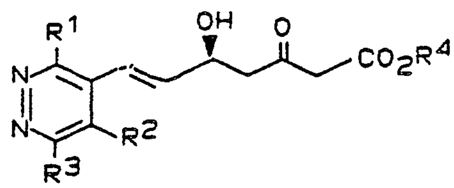

| Beispiel | $R^1$ | $R^2$ | $R^3$ | $R^4$ | Molmasse |
|---|---|---|---|---|---|
| 11 b | $CH_3$ | 4-$FC_6H_4$ | $C_6H_5$ | $C_2H_5$ | $C_{26}H_{25}FN_2O_4$ 448[$M^+$] |
| 11 c | $CH_3$ | $C_6H_5$ | $C_6H_5$ | $C_2H_5$ | $C_{26}H_{26}N_2O_4$ 430[$M^+$] |
| 11 d | i-$C_3H_7$ | $CH_3$ | $C_6H_5$ | $C_2H_5$ | $C_{23}H_{28}N_2O_4$ 396[$M^+$] |
| 11 e | i-$C_3H_7$ | 4-$FC_6H_4$ | i-$C_3H_7$ | $C_2H_5$ | $C_{25}H_{31}FN_2O_4$ 442[$M^+$] |
| 11 f | i-$C_3H_7$ | 4-$FC_6H_4$ | c-$C_6H_{11}$ | $C_2H_5$ | $C_{28}H_{35}FN_2O_4$ 482[$M^+$] |
| 11 g | 4-$FC_6H_4$ | i-$C_3H_7$ | $C_6H_5$ | $C_2H_5$ | $C_{28}H_{29}FN_2O_4$ 476[$M^+$] |
| 11 h | 4-$FC_6H_4$ | i-$C_3H_7$ | i-$C_3H_7$ | $C_2H_5$ | $C_{25}H_{31}FN_2O_4$ 442[$M^+$] |
| 11 i | t-$C_4H_9$ | 4-$FC_6H_4$ | $C_6H_5$ | $C_2H_5$ | $C_{29}H_{31}FN_2O_4$ 490[$M^+$] |
| 11 k | i-$C_3H_7$ | 4-$C_6H_4CH_3$ | 4-$C_6H_4CH_3$ | $C_2H_5$ | $C_{30}H_{34}N_2O_4$ 486[$M^+$] |

Fortsetzung Tabelle 11

| | | | | | |
|---|---|---|---|---|---|
| 11 l | i-$C_3H_7$ | 4-$C_6H_4OCH_3$ | 4-$C_6H_4OCH_3$ | $C_2H_5$ | $C_{30}H_{34}N_2O_6$ 518 [$M^+$] |
| 11 m | $CH_3$ | 4-$FC_6H_4$ | 4-$FC_6H_4$ | $C_2H_5$ | $C_{26}H_{24}F_2N_2O_4$ 466 [$M^+$] |

**Beispiel 12:**

Allgemeine Vorschrift zur Darstellung der Verbindungen der allgemeinen Formel I

Beispiel 12 a ($R^1$ = i-$C_3H_7$, $R^2$ = 4-$FC_6H_4$, $R^3$ = 4-$FC_6H_4$, $R^4$ = $C_2H_5$, X-Y = CH=CH)

3,4-Di-(4-fluorphenyl)-6-isopropyl-5-(3,5-dihydroxy-6-heptensäureethylester)-pyridazin

140 mg (0,3 mMol) der Verbindung gemäß Beispiel II a werden unter Argon in 4 ml trockenem THF gelöst, mit 0,4 ml (0,4 mMol) Triethylboranlösung (THF) versetzt und 10 Minuten bei Raumtemperatur gerührt. Bei -70° C werden 21,4 mg (0,6 mMol) Natriumborhydrid fest zugegeben. Anschließend spritzt man langsam 3 ml Methanol zu. Nach 3 h bei -70° C gießt man die Mischung auf eine gekühlte Lösung von 0,5 ml 35 proz. Wasserstoffperoxid in 4,4 ml Wasser. Nach 5 Minuten wird mit Ethylacetat extrahiert (3 x). Die vereinigten organischen Phasen werden mit gesättigter Natriumhydrogencarbonatlösung (2 x) und gesättigter Kochsalzlösung (1 x) gewaschen und getrocknet ($MgSO_4$). Abziehen des Lösungsmittels im Vakuum und Flash-Chromatographie auf Kieselgel (Cyclohexan/Ethylacetat 1/1 + 0,5 % $Et_3N$) ergibt 91 mg, Beispiel 12 a

Fp.: 85° C

MS, $C_{28}H_{30}F_2N_2O_4$ = 496 ($M+H^+$)

$^1$H-NMR: δ/ppm = 1,29 (tr,J=7Hz,3H); 1,47 (d,6H); 1,43 (m,2H); 2,44 (m,2H); 3,50 (h,1H); 4,15 (m,1H); 4,20 (q,2H); 4,40 (m,1H); 5,46 (dd,$J_1$=16Hz, $J_2$=7Hz,1H); 6,50 (d,J=16Hz,1H); 6,87-7,34 (m,8H)

In Analogie zu Beispiel 12 a wurden die Beispiele der Tabelle 12 erhalten.

**Tabelle 12:**

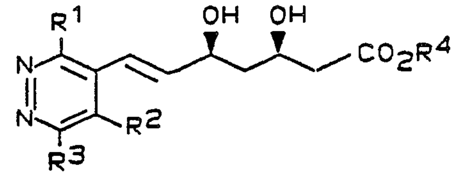

| Beispiel | $R^1$ | $R^2$ | $R^3$ | $R^4$ | Molmasse |
|---|---|---|---|---|---|
| 12 b | $CH_3$ | $4\text{-}FC_6H_4$ | $C_6H_5$ | $C_2H_5$ | $C_{26}H_{27}FN_2O_4$ 450[M⁺] |
| 12 c | $CH_3$ | $C_6H_5$ | $C_6H_5$ | $C_2H_5$ | $C_{26}H_{28}N_2O_4$ 432[M⁺] |
| 12 d | $i\text{-}C_3H_7$ | $CH_3$ | $C_6H_5$ | $C_2H_5$ | $C_{23}H_{30}N_2O_4$ 398[M⁺] |
| 12 e | $i\text{-}C_3H_7$ | $4\text{-}FC_6H_4$ | $i\text{-}C_3H_7$ | $C_2H_5$ | $C_{25}H_{33}FN_2O_4$ 444[M⁺] |
| 12 f | $i\text{-}C_3H_7$ | $4\text{-}FC_6H_4$ | $c\text{-}C_6H_{11}$ | $C_2H_5$ | $C_{28}H_{37}FN_2O_4$ 484[M⁺] |
| 12 g | $4\text{-}FC_6H_4$ | $i\text{-}C_3H_7$ | $C_6H_5$ | $C_2H_5$ | $C_{28}H_{31}FN_2O_4$ 478[M⁺] |
| 12 h | $4\text{-}FC_6H_4$ | $i\text{-}C_3H_7$ | $i\text{-}C_3H_7$ | $C_2H_5$ | $C_{25}H_{33}FN_2O_4$ 444[M⁺] |
| 12 i | $t\text{-}C_4H_9$ | $4\text{-}FC_6H_4$ | $C_6H_5$ | $C_2H_5$ | $C_{29}H_{33}FN_2O_4$ 492[M⁺] |
| 11 k | $i\text{-}C_3H_7$ | $4\text{-}C_6H_4CH_3$ | $4\text{-}C_6H_4CH_3$ | $C_2H_5$ | $C_{30}H_{36}N_2O_4$ 488[M⁺] |
| 12 l | $i\text{-}C_3H_7$ | $4\text{-}C_6H_4OCH_3$ | $4\text{-}C_6H_4OCH_3$ | $C_2H_5$ | $C_{30}H_{36}N_2O_6$ 520 [M⁺] |
| 12 m | $CH_3$ | $4\text{-}FC_6H_4$ | $4\text{-}FC_6H_4$ | $C_2H_5$ | $C_{26}H_{26}F_2N_2O_4$ 468 [M⁺] |

**Beispiel 13:**

Allgemeine Vorschrift zur Darstellung der Verbindungen der allgemeinen Formel I

Beispiel 13 a ($R^1$ = i-$C_3H_7$, $R^2$ = 4-$FC_6H_4$, $R^3$ = 4-$FC_6H_4$, $R^4$ = Na, X-Y = CH=CH)

3,4-Di-(4-fluorphenyl)-6-isopropyl-pyridazin-5-yl-(3,5-dihydroxy-6-heptensäure-natriumsalz)

42 mg (0,09 mMol) der Verbindung aus Beispiel 12 a werden in 2 ml Ethanol gelöst und mit 0,9 ml (0,09 mMol) 0,1 N Natronlauge versetzt. Nach 1 Stunde bei Raumtemperatur wird das Lösungsmittel unter mehrmaligen Zusatz von Toluol im Vakuum abgezogen. Der feste Rückstand wird mehrmals mit n-Pentan gewaschen und im Hochvakuum getrocknet.

In Analogie zu Beispiel 13 a wurden die Beispiele der Tabelle 13 erhalten.

**Tabelle 13:**

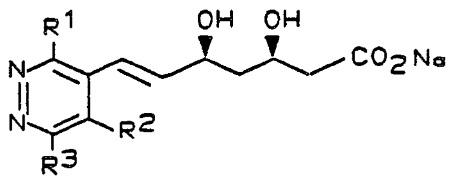

| Beispiel | $R^1$ | $R^2$ | $R^3$ | Molmasse |
|---|---|---|---|---|
| 13 b | $CH_3$ | 4-$FC_6H_4$ | $C_6H_5$ | $C_{24}H_{22}FN_2O_4Na$ 444[$M^+$] |
| 13 c | $CH_3$ | $C_6H_5$ | $C_6H_5$ | $C_{24}H_{23}N_2O_4Na$ 426[$M^+$] |
| 13 d | i-$C_3H_7$ | $CH_3$ | $C_6H_5$ | $C_{21}H_{25}N_2O_4Na$ 376[$M^+$] |
| 13 e | i-$C_3H_7$ | 4-$FC_6H_4$ | i-$C_3H_7$ | $C_{23}H_{28}FN_2O_4Na$ 438[$M^+$] |
| 13 f | i-$C_3H_7$ | 4-$FC_6H_4$ | c-$C_6H_{11}$ | $C_{26}H_{32}FN_2O_4Na$ 478[$M^+$] |

Fortsetzung Tabelle 13

| Beispiel | $R^1$ | $R^2$ | $R^3$ | Molmasse |
|---|---|---|---|---|
| 13 g | 4-$FC_6H_4$ | i-$C_3H_7$ | $C_6H_5$ | $C_{26}H_{26}FN_2O_4Na$ 472[$M^+$] |
| 13 h | 4-$FC_6H_4$ | i-$C_3H_7$ | i-$C_3H_7$ | $C_{23}H_{28}FN_2O_4Na$ 438[$M^+$] |
| 13 i | t-$C_4H_9$ | 4-$FC_6H_4$ | $C_6H_5$ | $C_{27}H_{28}FN_2O_4Na$ 486[$M^+$] |
| 13 k | i-$C_3H_7$ | 4-$C_6H_4CH_3$ | 4-$C_6H_4CH_3$ | $C_{28}H_{31}N_2O_4Na$ 482[$M^+$] |
| 13 l | i-$C_3H_7$ | 4-$C_6H_4OCH_3$ | 4-$C_6H_4OCH_3$ | $C_{28}H_{31}N_2O_6Na$ 514 [$M^+$] |
| 13 m | $CH_3$ | 4-$FC_6H_4$ | 4-$FC_6H_4$ | $C_{24}H_{21}F_2N_2O_4Na$ 462 [$M^+$] |

**Beispiel 14:**

Allgemeine Vorschrift zur Darstellung der Verbindungen der allgemeinen Formel I

Beispiel 14 a ($R^1$ = i-$C_3H_7$, $R^2$ = 4-$FC_6H_4$, $R^3$ = 4-$FC_6H_4$, X-Y = CH=CH)

6-(2-(3,4-Di-(4-fluorphenyl)-6-isopropyl-pyridazin-5-yl)-ethenyl)-4-hydroxy-3,4,5,6-tetrahydro-2H-pyran-2-on

10 mg (0,02 mMol) der Verbindung aus Beispiel 12 a werden mit 9,3 µl (0,1 mMol) Trifluoressigsäure in 1 ml absolutem Dichlormethan 12 Stunden bei Raumtemperatur gerührt. Die Lösung wird eingeengt und im Hochvakuum getrocknet. Nach Flash-Chromatographie auf Kieselgel (Ethylacetat) ergeben sich 9 mg der Titelverbindung als farbloses Öl.
MS, $C_{26}H_{24}F_2N_2O_3$ = 450 ($M^+$)
$^1$H-NMR: δ/ppm = 1,44 (d,J=7Hz,6H); 1,52-1,93 (m,3H); 2,61-2,84 (m,2H); 3,45 (h,J=7Hz,1H); 5,20 (m,1H); 5,50 (dd,$J_1$=16Hz, J2=7Hz,1H); 6,52 (d,J=16Hz,1H); 7,00-7,41(m,8H).

In Analogie zu Beispiel 14 a wurden die Beispiele der Tabelle 14 erhalten.

**Tabelle 14:**

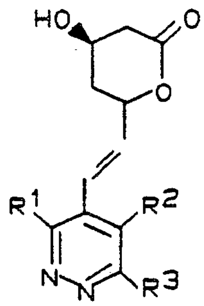

| Beispiel | $R^1$ | $R^2$ | $R^3$ | Molmasse |
|---|---|---|---|---|
| 14 b | $CH_3$ | $4\text{-}FC_6H_4$ | $C_6H_5$ | $C_{24}H_{21}FN_2O_3$ 404[$M^+$] |
| 14 c | $CH_3$ | $C_6H_5$ | $C_6H_5$ | $C_{24}H_{22}N_2O_3$ 386[$M^+$] |
| 14 d | $i\text{-}C_3H_7$ | $CH_3$ | $C_6H_5$ | $C_{21}H_{24}N_2O_3$ 352[$M^+$] |
| 14 e | $i\text{-}C_3H_7$ | $4\text{-}FC_6H_4$ | $i\text{-}C_3H_7$ | $C_{23}H_{27}FN_2O_3$ 398[$M^+$] |
| 14 f | $i\text{-}C_3H_7$ | $4\text{-}FC_6H_4$ | $c\text{-}C_6H_{11}$ | $C_{26}H_{31}FN_2O_3$ 438[$M^+$] |
| 14 g | $4\text{-}FC_6H_4$ | $i\text{-}C_3H_7$ | $C_6H_5$ | $C_{26}H_{25}FN_2O_3$ 432[$M^+$] |
| 14 h | $4\text{-}FC_6H_4$ | $i\text{-}C_3H_7$ | $i\text{-}C_3H_7$ | $C_{23}H_{27}FN_2O_3$ 398[$M^+$] |
| 14 i | $t\text{-}C_4H_9$ | $4\text{-}FC_6H_4$ | $C_6H_5$ | $C_{27}H_{27}FN_2O_3$ 446[$M^+$] |
| 14 k | $i\text{-}C_3H_7$ | $4\text{-}C_6H_4CH_3$ | $4\text{-}C_6H_4CH_3$ | $C_{28}H_{30}N_2O_3$ 442[$M^+$] |
| 14 l | $i\text{-}C_3H_7$ | $4\text{-}C_6H_4OCH_3$ | $4\text{-}C_6H_4OCH_3$ | $C_{28}H_{30}N_2O_5$ 474 [$M^+$] |
| 14 m | $CH_3$ | $4\text{-}FC_6H_4$ | $4\text{-}FC_6H_4$ | $C_{24}H_{20}F_2N_2O_3$ 422 [$M^+$] |

NMR-Spektren von Verbindungen gemäß Beispiel 14:

Beispiel 14 b:

$^1$H-NMR: δ/ppm = 1,54-1,90 (m, 3H); 2,65-2,80 (m, 2H); 2,72 (s, 3H); 5,21 (m, 1H); 5,52 (dd,$J_1$ = 16 Hz, $J_2$ = 7Hz, 1H); 6,50 (d, J = 16Hz, 1H); 7,0-7,4 (m, 10H)

Beispiel 14 l

$^1$H-NMR: δ/ppm = 1,42 (d, J=7Hz, 6H); 1,53-1,93 (m, 3H); 2,61-2,84 (m, 2H); 3,44 (h, J=7Hz, 1H); 3,90 (s, 6H); 5,21 (m, 1H); 5,52 (dd, $J_1$ = 16Hz, $J_2$=7Hz, 1H); 6,52 (d, J=16Hz; 1H); 7,0-7,4 (m, 8H).

Beispiel 14 m

$^1$H-NMR: δ/ppm = 1,53-1,89 (m, 3H); 2,63-2,75 (m,2H); 2,77 (s, 3H); 5,20 (m, 1H); 5,50 (dd, $J_1$ =16Hz, $J_2$=7Hz, 1H); 6,52 (d, J=16Hz, 1H); 7,0-7,4 (m, 8H).

**Beispiel 15:**

Allgemeine Vorschrift zur Darstellung der Verbindungen der allgemeinen Formel V (optisch aktiv)

Beispiel 15 a ($R^1$ = i=$C_3H_7$, $R^2$ = 4-$FC_6H_4$, $R^3$ = 4-$FC_6H_4$, $R^5$ = $CH(C_6H_5)-C(OH)(C_6H_5)_2$, X-Y = CH=CH)

3,4-Di-(4-fluorphenyl)-6-isopropyl-pyridazin-5-{3(S)-hydroxy-4-(E)-pentensäure-((S)-(-)-2-hydroxy-1,2,2-triphenylethyl)ester}

Zu einer Lösung von Diisopropylamin (33.6 ml; 240 mmol) in 400 ml absolutem THF tropft man bei -78°C langsam eine 1,6 M-Lösung von n-Butyllithium in Hexan (150 ml; 240 mmol) zu. Diese Lösung wird 30 Minuten bie 0°C gerührt, anschließend wird bei -78°C das (S)-(-)-2-hydroxy-1,2,2-triphenylacetat (35.5 g; 107 mmol) portionsweise zugegeben. Es wird 30 Minuten nachgerührt, auf 0°C aufgetaut, bis eine rote Lösung entsteht. Die Lösung wird wieder auf -78°C abgekühlt.
In einem weiteren Kolben werden 5.5 g (224 mmol) Magnesium-Späne mit 100 ml absolutem THF überlagert. Es wird nun Dibromethan (41.3 g; 220 mmol) so zugetropft, daß die Lösung leicht siedet. Nach beendeter Zugabe wird die Lösung auf -78°C abgekühlt und zu der oben hergestellten Lösung mittels einer Umdrücknadel langsam zugetropft. Jetzt wird die auf -78°C vorgekühlte Lösung von 39.2 g (107 mmol) Aldehyd aus Beispiel 10 a in 200 ml THF mit einer Umdrücknadel zu der oben hergestellten Lösung zugetropft und 1 Stunde bei -78°C gerührt. Das gekühlte Gemisch wird auf eine gesättigte Ammoniumchlorid-Lösung (500 ml) gegossen und 30 Minuten gerührt (pH 8, 0°C). Die organische Phase wird abgetrennt und die wäßrige Phase mit Ether extrahiert. Die vereinigten organi schen Phasen werden mit Natriumchlorid-Lösung gewaschen, getrocknet über Magnesiumsulfat. Nach Eindampfen des Lösungsmittels ergibt sich 48.2 g Beispiel 15 a als helle Kristalle.

Die Verbindung des Beispiels 15 a wird ohne weitere Reinigung zur nächsten Stufe eingesetzt.
Fp.: 80-85°C
MS, $C_{44}H_{38}F_2N_2O_4$ = 697 ($M+H^+$)
$^1$H-NMR: δ/ppm = 1.40 (d,J=7Kz,6H); 1.53 (s,1H); 2.04 (s,1H); 2.23 (d,2H); 3.38 (h,1H); 4.35 (m,1H); 5.35 (dd,$J_1$=16Hz,$J_2$=7Hz,1H); 6.37 (dd,$J_1$=16Hz,$J_2$=1.5Hz,1H); 6.70-7.55 (m,23H)

**Beispiel 16:**

Allgemeine Vorschrift zur Darstellung der Verbindungen der allgemeinen Formel V (optisch aktiv; Methylester)

Beispiel 16 a ($R^1$ = i-$C_3H_7$, $R^2$ = 4-$FC_6H_4$, $R^3$ = 4-$FC_6H_4$, $R^5$ = $CH_3$, X-Y = CH=CH)

3,4-Di-(4-fluorphenyl)-6-isopropyl-pyridazin-5-{3(S)-hydroxy-4(E)-pentensäuremethylester}

Zu einer Lösung von 2 g (2.9 mmol) Ester aus Beispiel 15 a in 100 ml absolutem Methanol wird eine Lösung aus 80.5 mg Natrium (3.5 mmol) in 25 ml absolutem Methanol bei 20 °C langsam zugetropft. Das Gemisch wird 1 Stunde bei Raumtemperatur gerührt, dann mit Eisessig neutralisiert und eingedampft. Der Rückstand wird mit je 100 ml Wasser und Ether aufgenommen; die Phasen werden getrennt und die organische Phase mit gesättigter Natriumhydrogencarbonat-Lösung gewaschen. Das Lösungsmittel wird nach Trocknen mit Magnesiumsulfat im Vakuum entfernt.
Der Rückstand wird chromatographiert (Cyclohexan, Ethylacetat 1/1) ind ergibt 1.0 g eines kristallinen Festkörpers.
Fp.: 125-127 °C
MS, $C_{25}H_{24}F_2N_2O_3$ = 439 ($M=H^+$)
$^1$H-NMR: δ/ppm = 1.44 (d,J=7Hz,6H); 1.58 (s,1H); 2.32 (m,2H); 3.43 (h,1H); 3.70 (s,3H); 4.50 (m,1H); 5.48 (dd,$J_1$=16Hz,$J_2$=7Hz,1H); 6.50 (dd,$J_1$=16Hz,$J_2$=1.5Hz,1H); 6.88-7.41 (m,8H)

**Beispiel 17:**

Allgemeine Vorschrift zur Darstellung der Verbindungen der allgemeinen Formel IV (optisch aktiv)

Beispiel 17 a ($R^1$ = i-$C_3H_7$, $R^2$=4-$FC_6H_4$, $R^3$ = 4-$FC_6H_4$, X-Y = CH=CH, $R^4$ = $^t$butyl)

3,4-Di-(4-fluorphenyl)-6-isopropyl-pyridazin-5-{5-(S)-hydroxy-3-oxo-6(E)-heptensäure-tert.-butylester}

Unter Argonatmosphäre wird zu 4 ml (28 mmol) Diisopropyolamin in 100 ml absolutem THF bei 0 °C 17.7 ml (28 mmol) n-Butyllithium (1.6 M-Lösung in Hexan) zugetropft und 30 Minuten bei 0 °C gerührt. Es wird auf -40 °C abgekühlt und 3.8 ml (28 mmol) Essigsäure-tert.-butylester zugetropft. Nach 1 Stunde bei -40 °C wird bei dieser Temperatur der Methylester aus Beispiel 16 a (3.1 g, 7.2 mmol), gelöst in 25 ml THF, zugetropft und 1 Stunde bei -20 °C gerührt. Die kalte Lösung wird auf gesättigte Ammoniumchlorid-Lösung gegossen, die organische Phase abgetrennt, anschließend mit Natriumchlorid-Lösung gewaschen, über Magnesium getrocknet und das Lösungsmittel im Vakuum eingedampft.
Der Rückstand wird an Kieselgel (Cyclohexan/Ethylacetat 1/1) chromatographiert und ergibt Beispiel 17 a als Farbloses Öl.
MS, $C_{30}H_{32}F_2N_2O_4$ = 523 ($M+H^+$)
$^1$H-NMR: δ/ppm = 1.48 (d+s,15H); 2.53 (m,2H); 3.32 (s,2H); 3.44 (h,1H); 4,57 (m,1H); 5.45 (dd,$J_1$=16Hz,$J_2$=7Hz,1H); 6.50 (dd,$J_1$=16Hz,$J_2$=1.5Hz,1H); 6.88-7.32 (m,8H)

**Beispiel 18:**

Allgemeine Vorschrift zur Darstellung der Verbindungen der allgemeinen Formel I (optisch aktiv)

Beispiel 18 a ($R^1$ = i-$C_3H_7$, $R^2$ = 4-$FC_6H_4$, $R^3$ = 4-$FC_6H_4$, $R^4$ = tert.-butyl, X-Y = CH=CH)

3,4-Di-(4-fluorphenyl)-6-isoproyl-pyridazin-5-{3(R),5(S)-dihydroxy-6(E)-heptensäure-tert.-butylester}

Die allgemeine Vorschrift entspricht der Vorschrift aus Beispiel 12 a
Öl
MS, $C_{30}H_{34}F_2N_2O_4$ = 525 ($M+H^+$)
$^1$H-NMR: δ/ppm = 1.45 (d+s,15H); 1.55 (m,2H); 2.32 (d,2H); 3.45 (h,1H); 3.64 (s,1H); 3.78 (s,1H); 4.08 (m,1H); 4.37 (m,1H); 5.39 (dd,1H); 6.44 (dd,1H); 6.85-7.27 (m,8H)

**Beispiel 19:**

Allgemeine Vorschrift zur Darstellung der Verbindungen der allgemeinen Formel I (optisch aktiv)

Beispiel 19 a ($R^1$ = i-$C_3H_7$, $R^2$ = 4-$FC_6H_4$, $R^3$ = 4-$FC_6H_4$, $R^4$ = Na, X-Y = CH=CH)

3,4-Di-(4-fluorphenyl)-6-isopropyl-pyridazin-5-{3(R),5(S)-dihydroxy-6(E)-heptensäure-natriumsalz}

Die allgemeine Vorschrift entspricht der Vorschrift aus Beispiel 13 a
Fp.: >230 °C
$^1$-H-NMR ($D_2O$): δ/ppm = 1.44 (d,6H); 1.62 (m,2H); 2.28 (d,2H); 3.60 (h,1H); 4.33 (m,1H); 4.80 (m,1H); 5.64 (dd,1H); 6.60 (dd,1H); 7.03-7.36 (m,8H)

## Ansprüche

1. 3,5-Dihydroxycarbonsäuren und deren Derivaten der allgemeinen Formel I

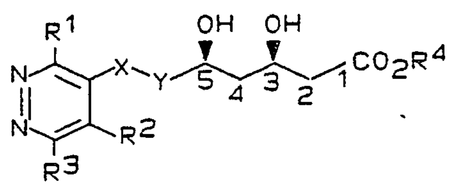

I

und die entsprechenden Laktone der Formel II

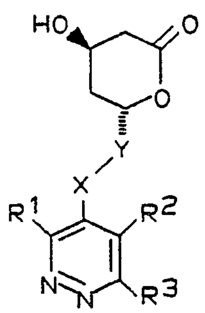

II

wobei in den allgemeinen Formeln I und II bedeuten
X-Y einen Rest der Formel -CH=CH- oder -$CH_2$-$CH_2$-
$R^1$, $R^2$, $R^3$ unabhängig voneinander Wasserstoff, einen gesättigten oder ungesättigten, geradkettigen oder verzweigten Kohlenwasserstoffrest mit bis zu 6 C-Atomen, der gegebenenfalls am terminalen Kohlenstoff durch einen gesättigten oder ungesättigten, cyclischen Kohlenwasserstoffrest mit 3 bis 6 C-Atomen, substituiert sein kann,
einen cyclischen gesättigten oder bis zu zweifach ungesättigten Kohlenwasserstoffrest mit 3 bis 7 C-Atomen, einen aromatischen Rest ausgewählt aus der Gruppe Phenyl, Furyl, Thienyl, Pyridyl, der gegebenenfalls im Kern 1 bis 3 gleiche oder verschiedene Substituenten der folgenden Gruppen tragen kann:
Halogen, Trifluormethyl, Alkyl oder Alkenyl mit je bis zu 6 C-Atomen, Hydroxy, Alkoxy mit 1 bis 6 C-Atomen, Carboxy, Carbalkoxy mit 1 bis 6 C-Atomen im Alkoxyteil
$R^4$ Wasserstoff, einen geradkettigen oder verzweigten, gesättigten oder ungesättigten Kohlenwasserstoffrest mit bis zu 8 C-Atomen, Mono- oder Dihydroxyalkyl mit 1 bis 4 C-Atomen, einen Phenyl- oder Benzylrest, deren Kerne 1- bis 2-fach mit Halogen oder einem Alkylrest mit 1 bis 4 C-Atomen substituiert sein können, Alkalimetall oder ein Ammoniumion.

2. Verbindungen der allgemeinen Formeln I und II gemäß Anspruch 1, dadurch gekennzeichnet, daß $R^1$ und $R^2$ einen geraddkettigen oder verzweigten Alkylrest mit 1 bis 4 C-Atomen, einen Cycloalkylrest mit 5 bis 6 C-Atomen, einen Cycloalkylmethyl- oder Cycloalkenylmethylrest mit einer Ringgröße von 5 bis 6 C-Atomen, einen Phenylrest, der gegebenenfalls 1 bis 3 gleiche oder verschiedene Substituenten der

folgenden Gruppen tragen kann Halogen, Trifluormethyl, Alkyl mit 1 bis 4 C-Atomen, Hydroxy, Alkoxy mit 1 bis 4 C-Atomen oder Carbalkoxy mit 1 bis 4 C-Atomen im Alkoxyteil, bedeutet,

$R^3$ Wasserstoff, einen geradkettigen oder verzweigten Alkyl-oder Alkenylrest mit bis zu 6 C-Atomen, einen Cycloalkyl-oder Cycloalkenylrest mit je 5 bis 6 C-Atomen, einen Phenyl- oder Pyridylrest, wobei die aromatischen Reste gegebenenfalls 1 bis 3 gleiche oder verschiedene Substituenten der folgenden Gruppen tragen können: Halogen, Alkyl mit 1 bis 4 C-Atomen, Hydroxy, Alkoxy mit 1 bis 4 C-Atomen oder Carbalkoxy mit 1 bis 4 C-Atomen im Alkoxyteil, bedeutet, und

$R^4$ Wasserstoff, Methyl, Ethyl, Isopropyl, Isobutyl, Benzyl, Natrium, Kalium, Ammonium ($NH_4$) oder Methyltris(hydroxymethyl)ammonium darstellt.

3. Verbindungen der allgemeinen Formeln I und II gemäß Anspruch 1, dadurch gekennzeichnet, daß

$R^1$ Methyl, Isopropyl, sek.-Butyl, tert.-Butyl, Cyclohexyl, Phenyl, 4-chlorphenyl, 4-Fluorphenyl, 4-Methoxyphenyl, 4-Fluor-3-methylphenyl, 3,5-Dimethylphenyl, Cyclohexylmethyl, 4-Trifluormethylphenyl ist

$R^2$ Methyl, Isopropyl, sek.-Butyl, tert.-Butyl, Cyclohexyl, Phenyl, 4-Chlorphenyl, 4-Fluorphenyl, 4-Methoxyphenyl, 4-Fluor-3-methylphenyl, 3,5-Dimethylphenyl, Cyclohexylmethyl, 4-Trifluormethylphenyl ist,

$R^3$ Wasserstoff, Methyl, Isopropyl, sek.-Butyl tert.-Butyl, Cyclohexyl, Phenyl, 4-Fluorphenyl, 2,5-dimethylphenyl, 3,5-Dimethylphenyl, 4-Trifluormethylphenyl ist, und

$R^4$ Wasserstoff, Methyl, Ethyl, Natrium, Kalium darstellt, herstellt.

4. Verbindung gemäß Anspruch 1, dadurch gekennzeichnet, daß die sie Formel I besitzen.

5. Verfahren zur Herstellung der Verbindung der allgemeinen Formeln I und II gemäß Anspruch 1, dadurch gekennzeichnet, daß man

a) entsprechend substituierte Aldehyde der Formel III

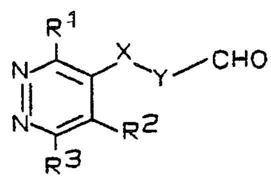

III

worin X-Y, $R^1$, $R^2$ und $R^3$ die angegebenen Bedeutungen haben, in die entsprechenden Hydroxyketoester der allgemeinen Formel IV überführt

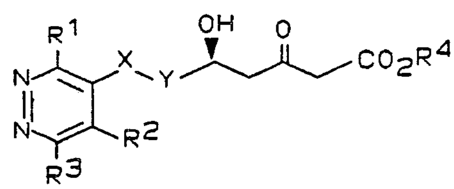

IV

worin X-Y, $R^1$, $R^2$ und $R^3$ die angegebenen Bedeutungen haben und $R^4$ Alkyl mit 1 bis 8 C-Atomen bedeutet,

b) die Hydroxyketoester der Formel IV in die entsprechenden 3,5-Dihydroxyverbindungen der Formel I

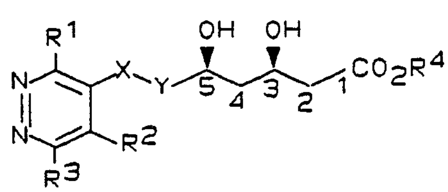

I

worin X-Y, $R^1$, $R^2$ und $R^3$ die zu Formel I angegebenen Bedeutungen haben, und $R^4$ Alkyl mit 1 bis 8 C-Atomen ist, überführt und eine erhaltene Verbindung gegebenenfalls verseift zu einer Verbindung der Formel I, worin $R^4$ ein Alkalimetallkation darstellt, daraus gegebenenfalls die freie Säure ($R^4$ = Wasserstoff) in Freiheit setzt und die freie Säure gegebenenfalls in Verbindungen der Formel I, worin $R^4$ die zu Formel I angegebenen Bedeutungen mit Ausnahme von Wasserstoff hat, überführt,

c) und eine erhaltene Verbindung der Formel I gegebenenfalls in ein Lakton der Formel II überführt,

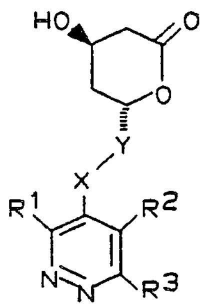

II

worin X-Y, $R^1$, $R^2$ und $R^3$ die angegebenen Bedeutungen haben

d) und eine erhaltene Verbindung, worin X-Y die CH=CH-Gruppe bedeutet, gegebenenfalls hydriert zu einer Verbindung, worin X-Y die $CH_2$-$CH_2$-Gruppe ist.

6. Pharmazeutische Präparate enthaltend eine Verbindung gemäß Anspruch 1.

7. Verwendung einer Verbindung gemäß Anspruch 1 zur Prophylaxe und Therapie der Hypercholesterinämie.

8. Verbindungen der Formel III

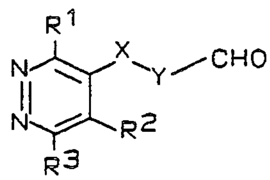

III

worin X-Y, $R^1$, $R^2$ und $R^3$ die in Anspruch 1 zu Formel I angegebenen Bedeutungen haben.

9. Verbindungen der Formel IV

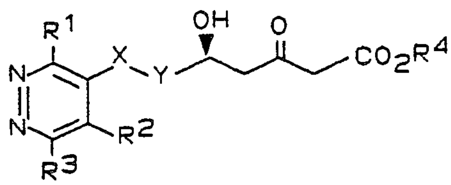

IV

worin X-Y, $R^1$, $R^2$ und $R^3$ die in Anspruch 1 zu Formel I angegebene Bedeutung haben und $R^4$ Alkyl mit 1 bis 8 C-Atomen ist.

10. Verbindungen der Formel VI

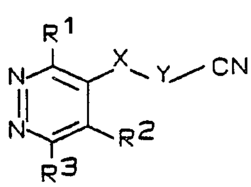

VI

der Formel VII

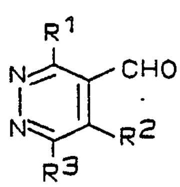

VII

der Formel VIII

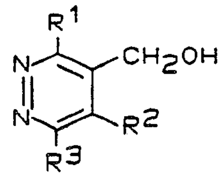

VIII

der Formel IX

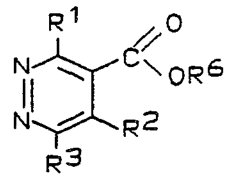

IX

worin $R^1$, $R^2$, $R^3$ und X-Y die in Anspruch 1 zu Formel I angegebenen Bedeutungen haben und $R^6$ eine Säureschutzgruppe ist.

Patentansprüche für folgenden Vertragsstaat: GR

1. Verfahren zur Herstellung von 3,5-Dihydroxycarbonsäuren und deren Derivaten der allgemeinen Formel I

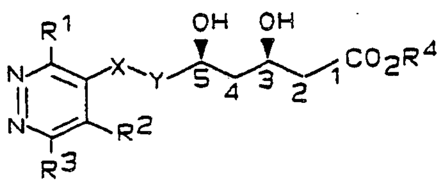

I

und der entsprechenden Laktone der Formel II

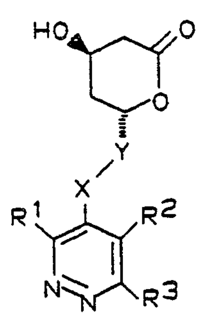

II

wobei in den allgemeinen Formeln I und II bedeuten
X-Y einen Rest der Formel -CH=CH- oder $-CH_2-CH_2-$
$R^1$, $R^2$, $R^3$ unabhängig voneinander Wasserstoff, einen gesättigten oder ungesättigten, geradkettigen oder verzweigten Kohlenwasserstoffrest mit bis zu 6 C-Atomen, der gegebenenfalls am terminalen Kohlenstoff durch einen gesättigten oder ungesättigten, cyclischen Kohlenwasserstoffrest mit 3 bis 6 C-Atomen, substituiert sein kann,
einen cyclischen gesättigten oder bis zu zweifach ungesättigten Kohlenwasserstoffrest mit 3 bis 7 C-Atomen, einen aromatischen Rest ausgewählt aus der Gruppe Phenyl, Furyl, Thienyl, Pyridyl, der gegebenenfalls im Kern 1 bis 3 gleiche oder verschiedene Substituenten der folgenden Gruppen tragen kann:

Halogen, Trifluormethyl, Alkyl oder Alkenyl mit je bis zu 6 C-Atomen, Hydroxy, Alkoxy mit 1 bis 6 C-Atomen, Carboxy, Carbalkoxy mit 1 bis 6 C-Atomen im Alkoxyteil
$R^4$ Wasserstoff, einen geradkettigen oder verzweigten, gesättigten oder ungesättigten Kohlenwasserstoffrest mit bis zu 8 C-Atomen, Mono- oder Dihydroxyalkyl mit 1 bis 4 C-Atomen, einen Phenyl- oder Benzylrest, deren Kerne 1- bis 2-fach mit Halogen oder einem Alkylrest mit 1 bis 4 C-Atomen substituiert sein können, Alkalimetall oder ein Ammoniumion, dadurch gekennzeichnet, daß man

a) entsprechend substituierte Aldehyde der Formel III

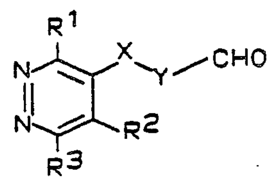

III

worin X-Y, $R^1$, $R^2$ und $R^3$ die angegebenen Bedeutungen haben, in die entsprechenden Hydroxyketoester der allgemeinen Formel IV überführt

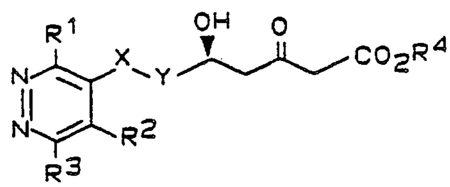

IV

worin X-Y, $R^1$, $R^2$ und $R^3$ die angegebenen Bedeutungen haben und $R^4$ Alkyl mit 1 bis 8 C-Atomen bedeutet,

b) die Hydroxyketoester der Formel IV in die entsprechenden 3,5-Dihydroxyverbindungen der Formel I

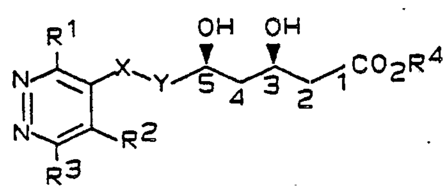

I

worin X-Y, $R^1$, $R^2$ und $R^3$ die zu Formel I angegebenen Bedeutungen haben, und $R^4$ Alkyl mit 1 bis 8 C-Atomen ist, überführt und eine erhaltene Verbindung gegebenenfalls verseift zu einer Verbindung der Formel I, worin $R^4$ ein Alkalimetallkation darstellt, daraus gegebenenfalls die freie Säure ($R^4$ = Wasserstoff) in Freiheit setzt und die freie Säure gegebenenfalls in Verbindungen der Formel I, worin $R^4$ die zu Formel I angegebenen Bedeutungen mit Ausnahme von Wasserstoff hat, überführt,

c) und eine erhaltene Verbindung der Formel I gegebenenfalls in ein Lakton der Formel II überführt,

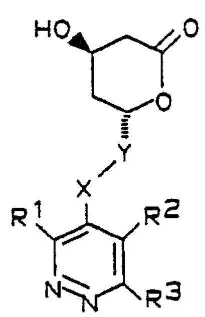

II

worin X-Y, $R^1$, $R^2$ und $R^3$ die angegebenen Bedeutungen haben

d) und eine erhaltene Verbindung, worin X-Y die CH=CH-Gruppe bedeutet, gegebenenfalls hydriert zu einer Verbindung, worin X-Y die $CH_2$-$CH_2$-Gruppe ist.

2. Verfahren gemäß Anspruch 1, dadurch gekennzeichnet, daß man eine Verbindung der allgemeinen Formel I oder II worin $R^1$ und $R^2$ einen geraddkettigen oder verzweigten Alkylrest mit 1 bis 4 C-Atomen, einen Cycloalkylrest mit 5 bis 6 C-Atomen, einen Cycloalkylmethyl- oder Cycloalkenylmethylrest mit einer Ringgröße von 5 bis 6 C-Atomen, eine Phenylrest, der gegebenenfalls 1 bis 3 gleiche oder verschiedene Substituenten der folgenden Gruppen tragen kann Halogen, Trifluormethyl, Alkyl mit 1 bis 4 C-Atomen, Hydroxy, Alkoxy mit 1 bis 4 C-Atomen oder Carbalkoxy mit 1 bis 4 C-Atomen im Alkoxyteil, bedeutet,
$R^3$ Wasserstoff, einen geradkettigen oder verzweigten Alkyl-oder Alkenylrest mit bis zu 6 C-Atomen, einen Cycloalkyl-oder Cycloalkenylrest mit je 5 bis 6 C-Atomen, einen Phenyl- oder Pyridylrest, wobei die aromatischen Reste gegebenenfalls 1 bis 3 gleiche oder verschiedene Substituenten der folgenden Gruppen tragen können: Halogen, Alkyl mit 1 bis 4 C-Atomen, Hydroxy, Alkoxy mit 1 bis 4 C-Atomen oder Carbalkoxy mit 1 bis 4 C-Atomen im Alkoxyteil, bedeutet, und
$R^4$ Wasserstoff, Methyl, Ethyl, Isopropyl, Isobutyl, Benzyl, Natrium, Kalium, Ammonium ($NH_4$) oder Methyltris(hydroxymethyl)ammonium darstellt, herstellt.

3. Verfahren gemäß Anspruch 1, dadurch gekennzeichnet, daß man eine Verbindung der allgemeinen Formel I oder II worin
$R^1$ Methyl, Isopropyl, sek.-Butyl, tert.-Butyl, Cyclohexyl, Phenyl, 4-Chlorphenyl, 4-Fluorphenyl, 4-Methoxyphenyl, 4-Fluor-3-methylphenyl, 3,5-Dimethylphenyl, Cyclohexylmethyl, 4-Trifluormethylphenyl ist
$R^2$ Methyl, Isopropyl, sek.-Butyl, tert.-Butyl, Cyclohexyl, Phenyl, 4-Chlorphenyl, 4-Fluorphenyl, 4-Methoxyphenyl, 4-Fluor-3-methylphenyl, 3,5-Dimethylphenyl, Cyclohexylmethyl, 4-Trifluormethylphenyl ist,
$R^3$ Wasserstoff, Methyl, Isopropyl, sek.-Butyl tert.-Butyl, Cyclohexyl, Phenyl, 4-Fluorphenyl, 2,5-Dimethylphenyl, 3,5-Dimethylphenyl, 4-Trifluormethylphenyl ist, und
$R^4$ Wasserstoff, Methyl, Ethyl, Natrium, Kalium darstellt, herstellt.

4. Verfahren gemäß Anspruch 1, dadurch gekennzeichnet, daß man eine Verbindung der Formel I herstellt.

5. Verfahren zur Herstellung eines pharmazeutischen Präparates dadurch gekennzeichnet, daß man eine Verbindung erhältlich gemäß Anspruch 1 in eine geeignete Darreichungsform bringt.

6. Verbindung der Formel III

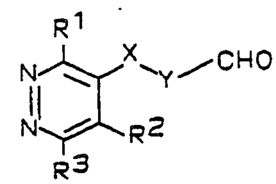

III

worin X-Y, $R^1$, $R^2$ und $R^3$ die in Anspruch 1 zu Formel I angegebenen Bedeutungen haben.

7. Verbindung der Formel IV

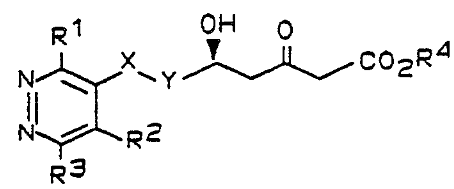

IV

worin X-Y, $R^1$, $R^2$ und $R^3$ die in Anspruch 1 zu Formel I angegebenen Bedeutung haben und $R^4$ Alkyl mit 1 bis 8 C-Atomen is.

8. Verbindungen der Formel VI

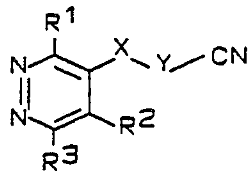

VI

der Formel VII

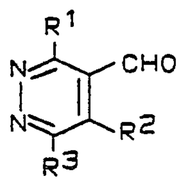

VII

der Formel VIII

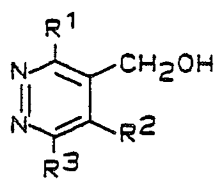

VIII

der Formel IX

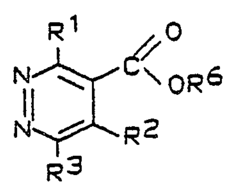

IX

worin $R^1$, $R^2$, $R^3$ und X-Y die in Anspruch 1 zu Formel I angegebenen Bedeutungen haben und $R^6$ eine Säureschutzgruppe ist.

Patentansprüche für folgenden Vertragsstaat: ES

1. Verfahren zur Herstellung von 3,5-Dihydroxycarbonsäuren und deren Derivaten der allgemeinen Formel I

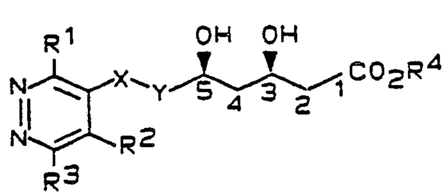

I

und der entsprechenden Laktone der Formel II

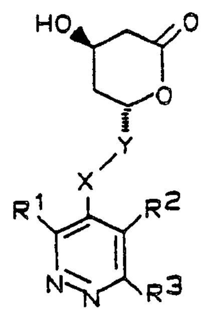

II

wobei in den allgemeinen Formeln I und II bedeuten
X-Y einen Rest der Formel -CH=CH- oder $-CH_2-CH_2-$
$R^1$, $R^2$, $R^3$ unabhängig voneinander Wasserstoff, einen gesättigten oder ungesättigten, geradkettigen oder verzweigten Kohlenwasserstoffrest mit bis zu 6 C-Atomen, der gegebenenfalls am terminalen Kohlenstoff durch einen gesättigten oder ungesättigten, cyclischen Kohlenwasserstoffrest mit 3 bis 6 C-Atomen, substituiert sein kann,
einen cyclischen gesättigten oder bis zu zweifach ungesättigten Kohlenwasserstoffrest mit 3 bis 7 C-Atomen, einen aromatischen Rest ausgewählt aus der Gruppe Phenyl, Furyl, Thienyl, Pyridyl, der gegebenenfalls im Kern 1 bis 3 gleiche oder verschiedene Substituenten der folgenden Gruppen tragen kann:
Halogen, Trifluormethyl, Alkyl oder Alkenyl mit je bis zu 6 C-Atomen, Hydroxy, Alkoxy mit 1 bis 6 C-Atomen, Carboxy, Carbalkoxy mit 1 bis 6 C-Atomen im Alkoxyteil
$R^4$ Wässerstoff, einen geradkettigen oder verzweigten, gesättigten oder ungesättigten Kohlenwasserstoffrest mit bis zu 8 C-Atomen, Mono- oder Dihydroxyalkyl mit 1 bis 4 C-Atomen, einen Phenyl- oder Benzylrestl, deren Kerne 1- bis 2-fach mit Halogen oder einem Alkylrest mit 1 bis 4 C-Atomen substituiert sein können, Alkalimetall oder ein Ammoniumion, dadurch gekennzeichnet, daß man

a) entsprechend substituierte Aldehyde der Formel III

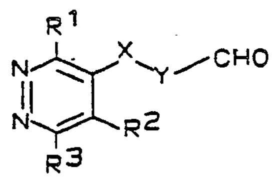

III

worin X-Y, $R^1$, $R^2$ und $R^3$ die angegebenen Bedeutungen haben, in die entsprechenden Hydroxyketoester der allgemeinen Formel IV überführt

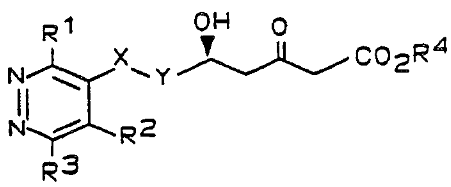

IV

worin X-Y, $R^1$, $R^2$ und $R^3$ die angegebenen Bedeutungen haben und $R^4$ Alkyl mit 1 bis 8 C-Atomen bedeutet,

b) die Hydroxyketoester der Formel IV in die entsprechenden 3,5-Dihydroxyverbindungen der Formel I

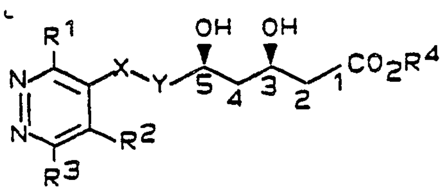

I

worin X-Y, $R^1$, $R^2$ und $R^3$ die zu Formel I angegebenen Bedeutungen haben, und $R^4$ Alkyl mit 1 bis 8 C-Atomen ist, überführt und eine erhaltene Verbindung gegebenenfalls verseift zu einer Verbindung der Formel I, worin $R^4$ ein Alkalimetallkation darstellt, daraus gegebenenfalls die freie Säure ($R^4$ = Wasserstoff) in Freiheit setzt und die freie Säure gegebenenfalls in Verbindungen der Formel I, worin $R^4$ die zu Formel I angegebenen Bedeutungen mit Ausnahme von Wasserstoff hat, überführt,

c) une eine erhaltene Verbindung der Formel I gegebenenfalls in ein Lakton der Formel II überführt,

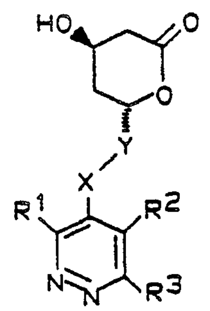

II

worin X-Y, $R^1$, $R^2$ und $R^3$ die angegebenen Bedeutungen haben

d) und eine erhaltene Verbindung, worin X-Y die CH=CH-Gruppe bedeutet, gegebenenfalls hydriert zu einer Verbindung, worin X-Y die $CH_2$-$CH_2$-Gruppe ist.

2. Verfahren gemäß Anspruch 1, dadurch gekennzeichnet, daß man eine Verbindung der allgemeinen Formel I oder II worin $R^1$ und $R^2$ einen geraddkettigen oder verzweigten Alkylrest mit 1 bis 4 C-Atomen, eine Cycloalkylrest mit 5 bis 6 C-Atomen, einen Cycloalkylmethyl- oder Cycloalkenylmethylrest mit einer Ringgröße von 5 bis 5 C-Atomen, einen Phenylrest, der gegebenenfalls 1 bis 3 gleiche oder verschiedene Substituenten der folgenden Gruppen tragen kann Halogen, Trifluormethyl, Alkyl mit 1 bis 4 C-Atomen, Hydroxy, Alkoxy mit 1 bis 4 C-Atomen oder Carbalkoxy mit 1 bis 4 C-Atomen im Alkoxyteil bedeutet,
$R^3$ Wasserstoff, einen geradkettigen oder verzweigten Alkyl-oder Alkenylrest mit bis zu 6 C-Atomen, einen Cycloalkyl-oder Cycloalkenylrest mit je 5 bis 6 C-Atomen, einen Phenyl- oder Pyridylrest, wobei die aromatischen Reste gegebenenfalls 1 bis 3 gleiche oder verschiedene Substituenten der folgenden Gruppen tragen können: Halogen, Alkyl mit 1 bis 4 C-Atomen, Hydroxy, Alkoxy mit 1 bis 4 C-Atomen oder Carbalkoxy mit 1 bis 4 C-Atomen im Alkoxyteil, bedeutet, und
$R^4$ Wasserstoff, Methyl, Ethyl, Isopropyl, Isobutyl, Benzyl, Natrium, Kalium, Ammonium ($NH_4$) oder Methyltris(hydroxymethyl)ammonium darstellt, herstellt.

3. Verfahren gemäß Anspruch 1, dadurch gekennzeichnet, daß man eine Verbindung der allgemeinen Formel I oder II worin $R^1$ Methyl, Isopropyl, sek.-Butyl, tert.-Butyl, Cyclohexyl, Phenyl, 4-Chlorphenyl, 4-Fluorphenyl, 4-Methoxyphenyl, 4-Fluor-3-methylphenyl, 3,5-Dimethylphenyl, Cyclohexylmethyl, 4-Trifluormethylphenyl ist
$R^2$ Methyl, Isopropyl, sek.-Butyl, tert.-Butyl, Cyclohexyl, Phenyl, 4-Chlorphenyl, 4-Fluorphenyl, 4-Methoxyphenyl, 4-Fluor-3-methylphenyl, 3,5-Dimethylphenyl, Cyclohexylmethyl, 4-Trifluormethylphenyl ist,
$R^3$ Wasserstoff, Methyl, Isopropyl, sek.-Butyl tert.-Butyl, Cyclohexyl, Phenyl, 4-Fluorphenyl, 2,5-Dimethylphenyl, 3,5-Dimethylphenyl, 4-Trifluormethylphenyl ist, und
$R^4$ Wasserstoff, Methyl, Ethyl, Natrium, Kalium darstellt, herstellt.

4. Verfahren gemäß Anspruch 1, dadurch gekennzeichnet, daß man eine Verbindung der Formel I herstellt.

5. Verfahren zur Herstellung eines pharmazeutischen Präparates dadurch gekennzeichnet, daß man eine Verbindung erhältlich gemäß Anspruch 1 in eine geeignete Darreichungsform bringt.

6. Verfahren zur Herstellung von Verbindungen der Formel III

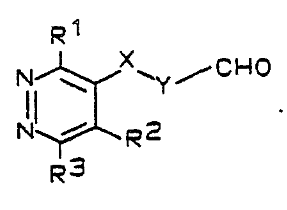

III

worin X-Y, $R^1$, $R^2$ und $R^3$ die in Anspruch 1 zu Formel I angegebenen Bedeutungen haben, dadurch

gekennzeichnet, daß man die entsprechenden Nitrile in die Aldehyde III überführt.

7. Verfahren gemäß Anspruch 1, dadurch gkennzeichnet, daß man ein Zwischenprodukt der Formel IV

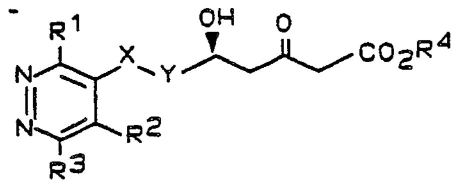

IV

worin X-Y, $R^1$, $R^2$ und $R^3$ die in Anspruch 1 zu Formel I angegebene Bedeutung haben und $R^4$ Alkyl mit 1 bis 8 C-Atomen ist, isoliert.

8. Verfahren gemäß Anspruch 1, dadurch gekennzeichnet, daß man ein Zwischenprodukt der Formel VI

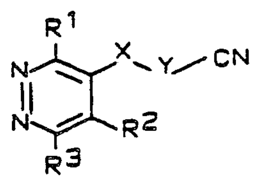

VI

der Formel VII

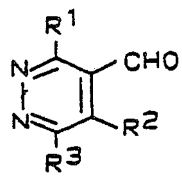

VII

der Formel VIII

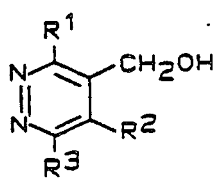

VIII

der Formel IX

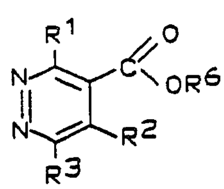

IX

worin $R^1$, $R^2$, $R^3$ und X-Y die in Anspruch 1 zu Formel I angegebenen Bedeutungen haben und $R^6$ eine Säureschutzgruppe ist, isoliert.